(19) Europäisches Patentamt
European Patent Office
Office européen des brevets

(11) EP 4 714 974 A1

(12) EUROPEAN PATENT APPLICATION
published in accordance with Art. 153(4) EPC

(43) Date of publication:
25.03.2026 Bulletin 2026/13

(21) Application number: 24806589.8

(22) Date of filing: 15.05.2024

(51) International Patent Classification (IPC):
C07K 16/28 (2006.01)    A61K 39/395 (2006.01)
A61P 35/00 (2006.01)    C07K 16/46 (2006.01)
C12N 15/13 (2006.01)    C12N 15/63 (2006.01)

(52) Cooperative Patent Classification (CPC):
A61K 39/395; A61P 35/00; C07K 16/00;
C07K 16/28; C07K 16/46; C12N 15/63

(86) International application number:
PCT/CN2024/093243

(87) International publication number:
WO 2024/235239 (21.11.2024 Gazette 2024/47)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 16.05.2023 CN 202310552022

(71) Applicant: Shanghai Qilu Pharmaceutical
Research and
Development Centre Ltd.
Shanghai 201203 (CN)

(72) Inventors:
• YANG, Liuqing
  Shanghai 201203 (CN)
• QIAN, Hongliang
  Shanghai 201203 (CN)
• LUO, Juan
  Shanghai 201203 (CN)

(74) Representative: Studio Torta S.p.A.
Via Viotti, 9
10121 Torino (IT)

Remarks:
The complete document including Reference
Table(s) and the Sequence Listing(s) can be
downloaded from the EPO website

(54) **BISPECIFIC ANTIGEN BINDING MOLECULE AND USE THEREOF**

(57) The present disclosure provides a form of a bispecific antigen binding molecule, and further provides a bispecific antigen binding molecule for GPRC5D and CD3 which is constructed on the basis of the form, a specific antigen binding molecule for GPRC5D or an antigen binding fragment thereof, a pharmaceutical composition containing the specific antigen binding molecule or the antigen binding fragment thereof, and a related use thereof in treatment of tumors.

Bispecific antibody 1    Bispecific antibody 2

Anti-GPRC5D full-length antibody Fab fragment

Anti-GPRC5D antibody heavy chain variable region

Anti-GPRC5D antibody light chain variable region

Anti-CD3 antibody light chain variable region

Anti-CD3 antibody heavy chain variable region

Fc domains with "knob" and "hole" structures

FIG. 1

**Description**

CROSS-REFERENCE TO RELATED APPLICATION

The present application claims priority and benefit to the Chinese Patent Application No. 202310552022.8 filed on May 16, 2023 and entitled "BISPECIFIC ANTIGEN BINDING MOLECULE AND USE THEREOF", the content of which is incorporated herein by reference in its entirety.

TECHNICAL FIELD

The present disclosure pertains to the field of immunology and relates to a bispecific antigen-binding molecule. The present disclosure also relates to an associated coding nucleic acid, vector, host cell, and drug, as well as associated use in cancer treatment.

BACKGROUND

Multiple myeloma (MM) is a hematologic malignancy and the second most common hematological malignancy after non-Hodgkin lymphoma, predominantly affecting middle-aged and elderly individuals. Multiple myeloma can damage bones, the immune system, kidneys, and red blood cell counts, often leading to extensive bone destruction accompanied by osteolytic lesions, osteopenia, and pathological fractures. According to statistics, the number of multiple myeloma patients in China increased from 69800 in 2016 to 101900 in 2019. With accelerated aging, this number is projected to rise to 167200 by 2024 and further to 266300 by 2030. Advances in diagnostic and therapeutic methods have also revealed younger cases of the disease. Multiple myeloma is highly prone to recurrence, with a median survival time of 24-36 months in Chinese patients and a five-year survival rate of only about 24%.

Like most cancer treatment methods, the standard treatment for multiple myeloma is chemotherapy, commonly using drugs such as melphalan. However, such drugs often causes myelosuppression, and their efficacy remains suboptimal, with a complete response rate of only about 5% after the treatment. These treatments are primarily used to relieve the disease condition. In recent years, with the progress of medicine, the development of targeting drugs such as proteasome inhibitors, immunomodulators, and antibody drugs has revolutionized the treatment of multiple myeloma. Compared with the original treatment regimens, targeting drugs have significantly increased the survival rate of multiple myeloma patients and significantly improved the prognosis of the patients. However, multiple myeloma is still incurable, and most patients relapse after multiple lines of treatment. Therefore, more researchers and clinicians began to search for new targets for recurrent/refractory multiple myeloma.

GPRC5D refers to the G-protein coupled receptor family C group 5 member D. It is an orphan receptor and a 7-transmembrane protein. GPRC5D is specifically highly expressed in plasma cells of multiple myeloma and is present only in hair follicles among normal tissues. The antibody and the CAR-T cell therapy targeting GPRC5D showed promising preclinical effects, and animals did not experience hair loss in the experiment. Moreover, the expression of GPRC5D is not limited by that of BCMA, and in a tumor recurrence model caused by BCMA antigen loss, the GPRC5D-targeted CAR-T therapy can overcome tumor escape. The results of these studies all indicate that GPRC5D is an ideal target in the clinic.

T cell-based therapies have shown significant anti-tumor effects in a number of animal models, and a number of T cell therapies have recently made significant progress in treating cancer indications. Therefore, harnessing the potential of T cells to develop novel T cell bispecific antibodies with high efficiency and low toxicity is particularly critical. Bispecific antibodies targeting the CD3 antigen are capable of spatially bringing T cells and tumor cells closer together and achieving a specific killing effect of the T cells on the tumor cells, and such antibodies are referred to as T cell-engagers (TCE bispecific antibodies). Among the bispecific antibodies in clinical stage, nearly half of the candidates target the CD3 antigen. During study on the safety and efficacy of the TCE bispecific antibodies, although certain progress has been made, many challenges still remain, such as how to balance anti-tumor activity and safety. The development of novel T cell bispecific antibodies with high efficiency and low toxicity is particularly critical.

Therefore, there is a need to develop new forms of bispecific antibodies and use them to further develop bispecific antibodies with high efficiency and low toxicity. The present disclosure provides an anti-GPRC5D/CD3 bispecific antibody, which is capable of recruiting T cells to a tumor site via a CD3 target, specifically killing tumor cells highly expressing GPRC5D, and enabling the cytotoxic effect of the T cells to be directed to cancer cells.

SUMMARY OF THE INVENTION

A first object of the present disclosure is to provide a bispecific antigen-binding molecule in a novel form, wherein a binding moiety specifically binding to a second antigen is sandwiched between a binding moiety specifically binding to a first antigen and an Fc structure. This form can reduce the exposure of the second antigen-binding moiety, thereby reducing the risk of the release of non-specific cytokines.

The bispecific antigen-binding molecule of the present disclosure comprises the following polypeptides:

a first polypeptide comprising: (i) an antigen-binding fragment Fab heavy chain domain capable of specifically binding to a first antigen, (ii) a single-chain variable fragment (scFv) domain capable of specifically binding to a second antigen, and (iii) a first Fc domain;

a second polypeptide comprising: an antigen-binding fragment Fab light chain domain capable of specifically binding to the first antigen;

a third polypeptide comprising: a second Fc domain.

The antigen-binding fragment Fab heavy chain domain of the first polypeptide and the antigen-binding fragment Fab light chain domain of the second polypeptide form a first binding site to the first antigen, the single-chain variable fragment (scFv) domain forms a second binding site to the second antigen, and the first Fc domain and the second Fc domain are associated with each other.

Optionally, the bispecific antigen-binding molecule further comprises a fourth polypeptide, wherein the fourth polypeptide comprises an antigen-binding fragment Fab light chain domain identical to the antigen-binding fragment Fab light chain domain of the second polypeptide and capable of specifically binding to the first antigen, and the third polypeptide further comprises an antigen-binding fragment Fab heavy chain domain capable of specifically binding to the first antigen, wherein the antigen-binding fragment Fab heavy chain domain is identical to the antigen-binding fragment Fab heavy chain domain of the first polypeptide and has its C-terminus linked to the N-terminus of the second Fc domain, and the antigen-binding fragment Fab heavy chain domain of the third polypeptide and the antigen-binding fragment Fab light chain domain of the fourth polypeptide form a third binding site to the first antigen.

In one embodiment, the single-chain variable fragment (scFv) domain comprises a heavy chain variable region and a light chain variable region.

Preferably, the heavy chain variable region of the single-chain variable fragment (scFv) domain is linked to the light chain variable region of the single-chain variable fragment (scFv) domain via a first linker, wherein the C-terminus of the heavy chain variable region of the single-chain variable fragment (scFv) domain is fused to the N-terminus of the first linker, and the C-terminus of the first linker is fused to the N-terminus of the light chain variable region of the single-chain variable fragment (scFv) domain.

More preferably, the first linker comprises the amino acid sequence $(G_4S)_n$, wherein n is any integer of 1-10.

In one embodiment, the first Fc domain comprises a first CH2 domain and a first CH3 domain of an immunoglobulin, wherein the C-terminus of the first CH2 domain is fused to the N-terminus of the first CH3 domain; the second Fc domain comprises a second CH2 domain and a second CH3 domain of an immunoglobulin, wherein the C-terminus of the second CH2 domain is fused to the N-terminus of the second CH3 domain.

Preferably, the first CH3 domain comprises a "knob" structure, and the second CH3 domain comprises a "hole" structure; more preferably, the "knob" structure comprises amino acid substitutions S354C and T366W, and the "hole" structure comprises amino acid substitutions Y349C, T366S, L368A, and Y407V.

Preferably, the Fc domain is derived from IgG1.

Preferably, the single-chain variable fragment (scFv) domain is linked to the first Fc domain via a second linker, wherein the C-terminus of the single-chain variable fragment (scFv) domain is fused to the N-terminus of the second linker, and the C-terminus of the second linker is fused to the N-terminus of the first Fc domain.

More preferably, the second linker comprises the amino acid sequence EPKSS.

In one embodiment, the antigen-binding fragment Fab heavy chain domain comprises a heavy chain variable region and a CH1 domain of an immunoglobulin, wherein the C-terminus of the Fab heavy chain variable region is fused to the N-terminus of the CH1 domain; the antigen-binding fragment Fab light chain domain comprises a light chain variable region and a light chain constant region of an immunoglobulin, wherein the C-terminus of the Fab light chain variable region is fused to the N-terminus of the light chain constant region.

Preferably, the antigen-binding fragment Fab heavy chain domain of the first polypeptide is linked to the single-chain variable fragment (scFv) domain via a third linker, wherein the C-terminus of the antigen-binding fragment Fab heavy chain domain is fused to the N-terminus of the third linker, and the C-terminus of the third linker is fused to the N-terminus of the single-chain variable fragment (scFv) domain.

More preferably, the third linker comprises the amino acid sequence $(G_4S)_n$, wherein n is any integer of 1-10.

Optionally, the C-terminus of the antigen-binding fragment Fab heavy chain domain of the third polypeptide is linked to the N-terminus of the second Fc domain via a fourth linker.

In one embodiment, the first polypeptide comprises the following structure: Fab heavy chain domain-third linker-scFv domain-second linker-first Fc domain, preferably comprises the following structure: Fab heavy chain variable region-Fab CH1-third linker-scFv heavy chain variable region-first linker-scFv light chain variable region-second linker-first CH2-first CH3; the second polypeptide comprises the following structure: Fab light chain variable region-light chain constant region; and the third polypeptide comprises the following structure: second CH2-second CH3.

In an optional embodiment, the third polypeptide comprises the following structure: Fab heavy chain domain-second Fc domain, preferably comprises the following structure: Fab heavy chain variable region-Fab CH1-second CH2-second CH3, and the fourth polypeptide comprises the following structure: Fab light chain variable region-light chain constant region.

In one embodiment, the Fc domain of the first polypeptide and/or the Fc domain of the third polypeptide comprise the following amino acid substitutions: L234A and/or L235A.

In one embodiment, the Fc domain of the third polypeptide comprises the following amino acid substitution: H435R.

In one embodiment, the second antigen is CD3, preferably CD3ε. Preferably, the single-chain variable fragment (scFv) domain comprises an HCDR1 set forth in SEQ ID NO: 19, an HCDR2 set forth in SEQ ID NO: 20, an HCDR3 set forth in SEQ ID NO: 21, an LCDR1 set forth in SEQ ID NO: 22, an LCDR2 set forth in SEQ ID NO: 23, and an LCDR3 set forth in SEQ ID NO: 24.

More preferably, the single-chain variable fragment (scFv) domain comprises a heavy chain variable region set forth in SEQ ID NO: 17 and a light chain variable region set forth in SEQ ID NO: 18.

More preferably, the single-chain variable fragment (scFv) domain comprises the amino acid sequence set forth in SEQ ID NO: 8.

In one embodiment, the first Fc domain comprises the amino acid sequence set forth in SEQ ID NO: 26, and the second Fc domain comprises the amino acid sequence set forth in SEQ ID NO: 4.

In one embodiment, the first antigen is GPRC5D. Preferably, the antigen-binding fragment Fab heavy chain domain comprises an HCDR1 set forth in SEQ ID NO: 11, an HCDR2 set forth in SEQ ID NO: 12, and an HCDR3 set forth in SEQ ID NO: 13, and/or, the antigen-binding fragment Fab light chain domain comprises an LCDR1 set forth in SEQ ID NO: 14, an LCDR2 set forth in SEQ ID NO: 15, and an LCDR3 set forth in SEQ ID NO: 16.

More preferably, the antigen-binding fragment Fab heavy chain domain comprises a heavy chain variable region set forth in SEQ ID NO: 9, and/or, the antigen-binding fragment Fab light chain domain comprises a light chain variable region set forth in SEQ ID NO: 10.

More preferably, the antigen-binding fragment Fab heavy chain domain comprises the amino acid sequence set forth in SEQ ID NO: 25; the antigen-binding fragment Fab light chain domain comprises the amino acid sequence set forth in SEQ ID NO: 3.

In one embodiment, the first polypeptide comprises the amino acid sequence set forth in SEQ ID NO: 1; the second polypeptide comprises the amino acid sequence set forth in SEQ ID NO: 3; the third polypeptide comprises the amino acid sequence set forth in SEQ ID NO: 4. In an optional embodiment, the first polypeptide comprises the amino acid sequence set forth in SEQ ID NO: 1; the second polypeptide and/or the fourth polypeptide comprise the amino acid sequence set forth in SEQ ID NO: 3; the third polypeptide comprises the amino acid sequence set forth in SEQ ID NO: 2.

A second object of the present disclosure is to provide an anti-GPRC5D (preferably anti-GPRC5D/CD3) bispecific antigen-binding molecule, which is capable of recruiting T cells to a tumor site by targeting an immune cell surface antigen (preferably a CD3 antigen) and specifically killing tumor cells highly expressing GPRC5D, with a relatively low level of *in vitro* cytokine release and good safety.

Based on this, the present disclosure provides a bispecific antigen-binding molecule binding to a specific epitope, wherein an epitope to which the bispecific antigen-binding molecule is capable of binding

is identical to or overlaps with an epitope targeted by an antibody comprising an HCDR1 set forth in SEQ ID NO: 11, an HCDR2 set forth in SEQ ID NO: 12, an HCDR3 set forth in SEQ ID NO: 13, an LCDR1 set forth in SEQ ID NO: 14, an LCDR2 set forth in SEQ ID NO: 15, and an LCDR3 set forth in SEQ ID NO: 16.

In one embodiment, an epitope to which the bispecific antigen-binding molecule is capable of binding

is identical to or overlaps with an epitope targeted by an antibody comprising the heavy chain variable region amino acid sequence set forth in SEQ ID NO: 9 and the light chain variable region amino acid sequence set forth in SEQ ID NO: 10.

In one embodiment, the bispecific antigen-binding molecule is capable of binding to CD3, preferably is capable of binding to CD3ε.

The present disclosure also provides a bispecific antigen-binding molecule comprising:

(A) a first binding moiety capable of specifically binding to GPRC5D; and

(B) a second binding moiety, wherein an antigen or epitope to which the second binding moiety specifically binds is different from that to which the first binding moiety binds;

the first binding moiety comprises:

an HCDR1 set forth in SEQ ID NO: 11, an HCDR2 set forth in SEQ ID NO: 12, an HCDR3 set forth in SEQ ID NO: 13, an LCDR1 set forth in SEQ ID NO: 14, an LCDR2 set forth in SEQ ID NO: 15, and an LCDR3 set forth in SEQ ID NO: 16.

In one embodiment, the first binding moiety comprises:

a heavy chain variable region set forth in SEQ ID NO: 9 and a light chain variable region set forth in SEQ ID NO: 10.

In one embodiment, the second binding moiety is capable of binding to CD3, preferably is capable of binding to CD3ε.

Another object of the present disclosure is to provide a specific antigen-binding molecule targeting GPRC5D or an antigen-binding fragment thereof, and the binding molecule or the antigen-binding fragment thereof is capable of specifically binding to GPRC5D but does not non-specifically bind to GPRC5A, a protein of the same family.

The GPRC5D-binding molecule or the antigen-binding fragment thereof comprises: the HCDR sequences of a heavy chain variable region set forth in SEQ ID NO: 9 and the LCDR sequences of a light chain variable region set forth in SEQ ID NO: 10; preferably, the GPRC5D-binding molecule or the antigen-binding fragment thereof comprises an HCDR1 set forth in SEQ ID NO: 11, an HCDR2 set forth in SEQ ID NO: 12, and an HCDR3 set forth in SEQ ID NO: 13, and/or, an LCDR1 set forth in SEQ ID NO: 14, an LCDR2 set forth in SEQ ID NO: 15, and an LCDR3 set forth in SEQ ID NO: 16; further preferably, the GPRC5D-binding molecule or the antigen-binding fragment thereof comprises the heavy chain variable region amino acid sequence set forth in SEQ ID NO: 9 and the light chain variable region amino acid sequence set forth in SEQ ID NO: 10.

In one embodiment, the GPRC5D-binding molecule or the antigen-binding fragment thereof comprises a heavy chain constant region and a light chain constant region; preferably, the GPRC5D-binding molecule or the antigen-binding fragment thereof comprises an IgG1 heavy chain constant region and a κ light chain constant region.

The present disclosure also provides a nucleic acid molecule, which encodes the bispecific antigen-binding molecule or the GPRC5D-binding molecule or the antigen-binding fragment thereof.

The present disclosure also provides an expression vector comprising the nucleic acid molecule.

The present disclosure also provides a host cell comprising the nucleic acid molecule or the expression vector; preferably, the host cell is a prokaryotic cell or a eukaryotic cell; the prokaryotic cell is preferably *Escherichia coli*; the eukaryotic cell is preferably a mammalian cell or a yeast; more preferably, the mammalian cell is a CHO cell, Expi293, or a HEK293 cell.

The present disclosure also provides a method for preparing the bispecific antigen-binding molecule or the GPRC5D-binding molecule or the antigen-binding fragment thereof, and the method comprises: culturing the host cell described above under suitable conditions.

The present disclosure also provides an antibody-drug conjugate formed by conjugating the bispecific antigen-binding molecule or the GPRC5D-binding molecule or the antigen-binding fragment thereof described above to an additional bioactive molecule; preferably, the additional bioactive molecule is a small-molecule drug; preferably, the bispecific antigen-binding molecule is linked to the additional bioactive molecule via a linker.

The present disclosure also provides a pharmaceutical composition comprising the bispecific antigen-binding molecule, the GPRC5D-binding molecule or the antigen-binding fragment thereof, the nucleic acid molecule, the expression vector, the host cell, and/or the antibody-drug conjugate described above.

In one embodiment, the pharmaceutical composition further comprises a pharmaceutically acceptable carrier.

In one embodiment, the pharmaceutical composition further comprises one or more additional therapeutic agents.

The present disclosure also provides use of the bispecific antigen-binding molecule, the nucleic acid molecule, the expression vector, the host cell, the antibody-drug conjugate, and/or the pharmaceutical composition described above in preparing a medicament for treating, alleviating, and/or preventing a tumor. Preferably, the tumor is a GPRC5D-positive tumor.

The present disclosure also provides a method for inducing death of a cell expressing GPRC5D *in vivo* or *in vitro,* comprising contacting the cell with the bispecific antigen-binding molecule, the GPRC5D-binding molecule or the antigen-binding fragment thereof, the nucleic acid molecule, the expression vector, the host cell, the antibody-drug conjugate, and/or the pharmaceutical composition described above, wherein, preferably, the cell expressing GPRC5D is a tumor cell.

The present disclosure also provides a method for treating a disease associated with GPRC5D expression in a subject, comprising administering to a subject in need thereof the bispecific antigen-binding molecule, the GPRC5D-binding molecule or the antigen-binding fragment thereof, the nucleic acid molecule, the expression vector, the host cell, the antibody-drug conjugate, and/or the pharmaceutical composition described above. Preferably, the disease is a tumor. Preferably, the subject suffers from recurrence or refractoriness following treatment with prior anti-cancer therapeutic agents.

Preferably, the tumor/tumor cell is selected from: lymphomas such as multiple myeloma, and metastatic cancers of the tumors described above.

In one embodiment, the method further comprises administering to the subject an additional therapeutic agent.

The present disclosure also provides the bispecific antigen-binding molecule, the GPRC5D-binding molecule or the antigen-binding fragment thereof, the nucleic acid molecule, the expression vector, the host cell, the antibody-drug conjugate, and/or the pharmaceutical composition described above, for use in therapy.

The substitutions referred to in the present disclosure are preferably represented by the EU numbering scheme.

The technical solution of the present disclosure has the following beneficial effects: it maintains a relatively strong *in vitro* tumor cell killing ability, while having relatively weak or no non-specific cytokine release *in vitro,* thereby ensuring good safety.

BRIEF DESCRIPTION OF THE DRAWINGS

The drawings further illustrate the novel features disclosed in this specification. The features and advantages disclosed in this specification will be better understood with reference to the drawings, but it should be understood that the drawings are merely used to illustrate the specific embodiments of the principles disclosed herein and are not intended to limit the scope of the appended claims.

FIG. 1 shows the structures of two bispecific antibodies constructed in the examples, which are bispecific antibody 1 and bispecific antibody 2, respectively.

FIG. 2A shows the binding of bispecific antibody 1 and bispecific antibody 2 to cells expressing hGPRC5D (NCI-H929 cells).

FIG. 2B shows the binding of bispecific antibody 1 and bispecific antibody 2 to cells expressing hGPRC5D (RPMI8226 cells).

FIG. 2C shows the binding of bispecific antibody 1 and bispecific antibody 2 to cells expressing hCD3 (Jurkat cells).

FIG. 3A shows the results of a T cell-dependent cell-mediated cytotoxicity (TDCC) experiment, with the target cells being NCI-H929.

FIG. 3B shows the results of a T cell-dependent cell-mediated cytotoxicity (TDCC) experiment, with the target cells being RPMI8226.

FIG. 3C shows the release of cytokine IFNγ under the condition of co-incubation of PBMCs and NCI-H929 tumor cells with the bispecific antibody molecules.

FIG. 3D shows the release of cytokine IFNγ under the condition of incubation of the bispecific antibody molecules with only PBMCs.

FIG. 3E shows the release of cytokine IL6 under the condition of co-incubation of PBMCs and NCI-H929 tumor cells with the bispecific antibody molecules.

FIG. 3F shows the release of cytokine IL6 under the condition of incubation of the bispecific antibody molecules with only PBMCs.

FIG. 4 shows the efficacy of the bispecific antibody molecules in a human immune cell reconstitution mouse model (with tumor cells being NCI-H929).

FIG. 5 shows the efficacy of the bispecific antibody molecules in a human immune cell reconstitution mouse model (with tumor cells being RPMI8226).

FIGs. 6A and 6B show the binding of the chimeric mouse hybridoma-derived anti-GPRC5D antibodies of the present disclosure to cells expressing GPRC5D (human GPRC5D-CHOK1 cell line).

FIGs. 6C and 6D show the binding of the chimeric mouse hybridoma-derived anti-GPRC5D antibodies of the present disclosure to cells expressing GPRC5D (cynomolgus monkey GPRC5D-CHOK1 cell line).

FIG. 7 shows that the chimeric mouse hybridoma-derived anti-GPRC5D antibodies of the present disclosure do not non-specifically bind to GPRC5A, a member of the same family.

FIGs. 8A and 8B show the binding of the humanized mouse hybridoma-derived anti-GPRC5D antibodies of the present disclosure to cells expressing GPRC5D (human GPRC5D-CHOK1 cell line).

FIGs. 8C and 8D show the binding of the humanized mouse hybridoma-derived anti-GPRC5D antibodies of the present disclosure to cells expressing GPRC5D (NCI-H929 tumor cells).

DETAILED DESCRIPTION OF THE INVENTION

## Terminology

All publications, patents, and patent applications referred to in the specification are incorporated herein by reference to the same extent as if each individual publication, patent, or patent application was specifically and individually incorporated herein by reference.

Before the present disclosure is described in detail below, it should be understood that the present disclosure is not limited to the particular methodology, protocols, and reagents described herein, as these may vary. It should also be understood that the terminology used herein is only intended to describe specific embodiments rather than to limit the scope of the present disclosure. Unless otherwise defined, any technical and scientific term used herein has the same meaning as commonly understood by those of ordinary skill in the art to which the present disclosure belongs.

Certain embodiments disclosed herein encompass numerical ranges, and certain aspects of the present disclosure may be described by means of ranges. Unless otherwise indicated, it should be understood that the numerical ranges or the description by means of ranges are merely for the purposes of brevity and convenience, and should not be construed as a strict definition of the scope of the present disclosure. Accordingly, the description by means of ranges should be considered to have specifically disclosed all the possible subranges as well as all the possible specific numerical points within that range, as if those subranges and numerical points were explicitly written out herein. The principle described above applies regardless of the extent of the numerical values. When a range description is present, the range includes range endpoints.

When referring to a measurable value such as an amount, temporal duration of time, the term "about" is meant to include a variation of $\pm 20\%$, or $\pm 10\%$ in some cases, or $\pm 5\%$ in some cases, or $\pm 1\%$ in some cases, or $\pm 0.1\%$ in some cases of the specified value.

The three-letter and single-letter codes for amino acid residues used herein are as described in J. Biol. Chem, 243, p3558 (1968).

The term "antibody" herein may include an intact antibody (e.g., a full-length monoclonal antibody) and any antigen-binding fragment (i.e., antigen-binding moiety) thereof or a single chain thereof, and may also include a product with antigen-specific binding ability formed by engineering (e.g., linking to other peptide fragments, rearrangement of functional units, etc.) on the basis of an intact antibody or an antigen-binding fragment thereof or a single chain thereof.

In one embodiment, an antibody typically refers to a Y-type tetrameric protein comprising two heavy (H) polypeptide chains and two light (L) polypeptide chains held together by covalent disulfide bonds and non-covalent interactions. Natural IgG antibodies have such a structure. Each light chain consists of one variable domain (VL) and one constant domain (CL). Each heavy chain comprises one variable domain (VH) and constant regions.

Five major classes of antibodies are known in the art: IgA, IgD, IgE, IgG, and IgM, with their corresponding heavy chain constant domains referred to as $\alpha$, $\delta$, $\varepsilon$, $\gamma$, and $\mu$, respectively. IgG and IgA may be further divided into different subclasses. For example, IgG may be divided into IgG1, IgG2, IgG3, and IgG4, and IgA may be divided into IgA1 and IgA2. The light chains of the antibodies from any vertebrate species can be classified into one of two distinct types, called $\kappa$ and $\lambda$, based on the amino acid sequences of their constant domains.

In the case of IgG, IgA, and IgD antibodies, the constant region comprises three domains referred to as CH1, CH2, and CH3 (IgM and IgE have a fourth domain, CH4). In IgG, IgA, and IgD classes, the CH1 and CH2 domains are separated by a flexible hinge region, and the hinge region is a proline- and cysteine-rich segment of variable length. Each class of antibodies further comprises inter-chain and intra-chain disulfide bonds formed by paired cysteine residues.

The term "variable region" or "variable domain" shows significant variation in amino acid composition from one antibody to another and is primarily responsible for antigen recognition and binding. The variable region of each light/heavy chain pair forms an antibody binding site, such that an intact IgG antibody has two binding sites (i.e., it is bivalent). The variable region domain of the heavy chain (VH) and the variable region domain of the light chain (VL) each comprise three regions with extreme variability, called hypervariable regions (HVRs), or more generally, complementarity determining regions (CDRs). VH and VL each have 4 framework regions (FRs), denoted as FR1, FR2, FR3, and FR4, respectively. Thus, CDR and FR sequences generally appear in the following sequence of a heavy chain variable domain (or a light chain variable domain): FR1-HCDR1 (LCDR1)-FR2-HCDR2 (LCDR2)-FR3-HCDR3 (LCDR3)-FR4.

The term "antibody fragment" comprises at least a portion of an intact antibody. As used herein, the "fragment" of an antibody molecule includes an "antigen-binding fragment" of an antibody, and the term "antigen-binding fragment" refers to a polypeptide fragment of an immunoglobulin or antibody specifically binding to or reacting with a selected antigen or an immunogenic determining moiety thereof, or a fusion protein product further derived from such a fragment, e.g., a single-chain variable fragment, an extracellular binding region in a chimeric antigen receptor, and the like. Exemplary antibody fragments or antigen-binding fragments thereof include, but are not limited to: variable light chain fragments, variable heavy chain fragments, Fab fragments, F(ab')$_2$ fragments, Fd fragments, Fv fragments, single-domain antibodies, linear antibodies, single-chain variable fragments (scFvs), bispecific antibodies or multispecific antibodies formed by antibody fragments, and the like.

The term "Fab" or "Fab fragment" refers to a monovalent antibody fragment consisting of the VH and CH1 domains of the heavy chain and the VL and CL domains of the light chain. The term "F(ab')$_2$" or "F(ab')$_2$ fragment" refers to a fragment comprising two Fab fragments and a hinge region, and is a bivalent antibody fragment.

The term "single-chain variable fragment" or "scFv" refers to a fusion protein comprising at least one antibody fragment comprising a variable region of a light chain and at least one antibody fragment comprising a variable region of a heavy chain, wherein the light and heavy chain variable regions are contiguously linked (e.g., via a synthetic linker, such as a short flexible polypeptide linker), and can be expressed as a single-chain polypeptide, and the scFv retains the specificity of the intact antibody from which it is derived. Unless specified, the scFv may comprise the VL and VH variable regions in any order (e.g., relative to the N-terminus and C-terminus of the polypeptide), and may comprise VL-linker-VH or VH-linker-VL.

The term "amino acid modification" (or "modified amino acid") includes amino acid substitutions, insertions, and/or deletions in a polypeptide sequence. The "amino acid substitution" or "substitution" herein means the replacement of an amino acid residue at a specific position in a parent polypeptide sequence with another amino acid residue. For example, the substitution S32A refers to the replacement of serine at position 32 with alanine.

When the variable regions in the present disclosure are used to prepare an antibody, a binding molecule, a bispecific binding molecule, or a multispecific binding molecule, the constant region is not particularly limited. A constant region known to those skilled in the art or a self-obtained constant region may be used, and amino acid mutations (e.g., mutations that improve or reduce binding to an Fc receptor or FcRn) may also be introduced into the constant region moiety.

The term "Fc" is used to define a C-terminus region of an immunoglobulin heavy chain, and the region comprises at least a portion of a constant region. The term includes Fc regions of natural sequences and variant Fc regions. Although the boundaries of the Fc region of an IgG heavy chain may vary slightly, the human IgG heavy chain Fc region is usually defined to extend from Cys226 or Pro230 to the carboxyl-terminus of the heavy chain. For example, the IgG Fc domain comprises IgG CH2 and IgG CH3 constant domains. Unless otherwise specified herein, the numbering of the amino acid residues in the Fc region or constant region conforms to the EU numbering scheme, also known as the EU index.

The bispecific antigen-binding molecule of the present disclosure may also comprise substitutions or modifications of the constant region (e.g., Fc), including but not limited to amino acid residue substitutions, mutations, and/or modifications, which result in compounds with preferred characteristics. These preferred characteristics include, but are not limited to: altered pharmacokinetics, increased serum half-life, improved binding affinity, reduced immunogenicity, increased production, altered Fc ligand binding to Fc receptors (FcRs), enhanced or reduced ADCC or CDC, altered glycosylation and/or disulfide bonds, and modified binding specificity. In certain aspects, an antibody variant comprises an Fc region with one or more amino acid substitutions that impair Fc$\gamma$R binding (e.g., substitutions at positions 234 and 235 of the Fc region). In one aspect, the substitutions are L234A and L235A.

The term "knob-into-hole" refers to a modification for promoting the association of two polypeptide chains of Fc, and it comprises a "knob" modification in one of the two polypeptide chains of the Fc and a "hole" modification in the other one of the two polypeptide chains of the Fc. The technique is documented in, e.g., US 5,731,168; US 7,695,936; Ridgway et al., Prot Eng, 9, 617-621 (1996) and Carter, J Immunol Meth, 248, 7-15 (2001). Generally, the method involves introducing a protuberance ("knob") at the interface of a first polypeptide chain and a corresponding cavity ("hole") at the interface of a second polypeptide chain, such that the protuberance can be positioned in the cavity so as to promote heterodimer formation and hinder homodimer formation. The protuberance is constructed by replacing a small amino acid side chain from the interface of the first polypeptide chain with a larger side chain (e.g., tyrosine or tryptophan). A complementary cavity having the same or similar size as the protuberance is created at the interface of the second polypeptide chain by replacing a large amino acid side chain with a smaller amino acid side chain (e.g., alanine or threonine).

Thus, in a specific embodiment, in the CH3 domain of the first polypeptide chain of the Fc domain of the bispecific antigen-binding molecule of the present disclosure, one amino acid residue is replaced with an amino acid residue having a larger side chain volume, thereby generating a protuberance within the CH3 domain of the first polypeptide chain, and the protuberance can be positioned in a cavity within the CH3 domain of the second polypeptide chain; and in the CH3 domain of the second polypeptide chain of the Fc domain, one amino acid residue is replaced with an amino acid residue having a smaller side chain volume, thereby generating the cavity within the CH3 domain of the second polypeptide chain, and the protuberance within the CH3 domain of the first polypeptide chain can be positioned in the cavity. Preferably, the amino acid residue having a larger side chain volume is selected from the following group: arginine (R), phenylalanine (F), tyrosine (Y), and tryptophan (W). Preferably, the amino acid residue having a smaller side chain volume is selected from the following group: alanine (A), serine (S), threonine (T), and valine (V).

The term "linker" refers to any tool for linking two different functional units (e.g., antigen-binding fragments). Types of linkers include, but are not limited to, chemical linkers and polypeptide linkers. The sequence of a polypeptide linker is not limited. Polypeptide linkers are preferably non-immunogenic and flexible, e.g., those comprising serine and glycine sequences. Linkers can be long or short, depending on the specific construct.

According to the present disclosure, the linker linking different functional units preferably comprises a flexible peptide linker, such as a glycine-serine peptide linker. In one embodiment, the linker comprises the amino acid sequence $(G_4S)_n$ or $(G_4S)_nA$, wherein n is any integer selected from 1-10. Preferably, the linker comprises the amino acid sequence $(G_4S)_3$ or $(G_4S)_3A$. The linker linking the VH and VL domains to form a VH-VL or VL-VH scFv domain preferably comprises a flexible peptide linker, such as a glycine-serine peptide linker. In one embodiment, the linker comprises the amino acid sequence $(G_4S)_n$ or $(G_4S)_nA$, wherein n is any integer selected from 1-10. Preferably, the linker comprises the amino acid sequence $(G_4S)_3$ or $(G_4S)$.

The "antibody" herein may be used in the broadest sense and may include, for example, polyclonal antibodies, monoclonal antibodies, chimeric antibodies, humanized and primatized antibodies, CDR-grafted antibodies, human antibodies (including human antibodies recombinantly produced), antibodies recombinantly produced, intracellular antibodies, multispecific antibodies, bispecific antibodies, monovalent antibodies, multivalent antibodies, anti-idiotypic antibodies, synthetic antibodies (including mutant proteins and variants thereof), and the like.

The term "monoclonal antibody" (or "mAb") refers to a substantially homogeneous antibody produced by a single cell clone and targeting only a particular antigen epitope. Monoclonal antibodies can be prepared using a variety of techniques known in the art, including hybridoma techniques, recombinant techniques, phage display techniques, transgenic animals, synthetic techniques, combinations of the techniques described above, or the like.

It should be noted that the CDRs and FRs of the variable regions of the antibodies and bispecific antigen-binding molecules of the present disclosure are defined according to the Kabat scheme. Other naming and numbering schemes, such as Chothia, IMGT, AHo, are also known to those skilled in the art. Therefore, based on the antibody sequence of the present disclosure, humanized antibodies comprising one or more CDRs derived according to any naming scheme all explicitly remain within the scope of the present disclosure.

The term "humanized antibody" refers to an antibody in which all or part of the amino acid residues except for the CDRs of a non-human antibody (e.g., a mouse antibody) are replaced with corresponding amino acid residues derived from human immunoglobulins. Small additions, deletions, insertions, substitutions, or modifications of amino acids are permissible only if they do not eliminate the ability of the antibody to bind to a specific antigen. "Humanized" antibodies retain antigen specificity similar to that of the original antibody.

The term "chimeric antibody" refers to an antibody in which the variable regions are derived from one species and the constant regions are derived from another species, e.g., an antibody in which the variable regions are derived from a mouse antibody and the constant regions are derived from a human antibody.

As used herein, "homologous" and "heterologous" are a group of relative concepts, and can mean that the sources of different elements in a construct are the same or different, or that after the construct is constructed, some of the elements that are originally from the same source, i.e., "homologous", are modified and different from the other original elements that have not been modified, thus becoming "heterologous".

The term "antigen" refers to a substance that is recognized and specifically bound by an antibody or an antigen-binding fragment. In a broad sense, the antigen may include any immunogenic fragment or determinant of a selected target, including a single epitope, a multiple epitope, a single domain, a multiple domain, an intact extracellular domain (ECD), or a protein. Peptides, proteins, glycoproteins, polysaccharides, and lipids, as well as portions thereof and combinations thereof, all can constitute antigens. Non-limiting exemplary antigens include tumor antigens or pathogen antigens and the like. "Antigen" may also refer to a molecule that induces an immune response. Any form of the antigen or cells or formulations containing the antigen can be used to generate antibodies having specificity to the antigenic determinant.

The term "epitope" or "antigenic determinant" refers to a site on an antigen to which an immunoglobulin or antibody specifically binds. An epitope may be formed by contiguous amino acid residues, or non-contiguous amino acid residues juxtaposed by tertiary folding of the protein. An epitope formed by contiguous amino acid residues is generally retained after exposure to a denaturing solvent, while an epitope formed by tertiary folding is generally lost after a denaturing solvent treatment. An epitope generally exists in a unique spatial conformation and comprises at least 3-15 amino acid residues.

The term "bispecific" means that an antigen-binding molecule is capable of specifically binding to two different antigenic determinants. The term "antigen-binding molecule" in its broadest sense refers to a molecule specifically binding to an antigenic determinant. Examples of antigen-binding molecules are immunoglobulins and derivatives thereof, such as fragments. The term "bispecific antigen-binding molecule" or "bispecific binding molecule" refers to a binding molecule (e.g., an antibody or a molecule comprising an antibody fragment) having specificity to two different antigens (or epitopes), particularly a bispecific antibody.

The term "specific binding" means that the binding is selective for antigens and can be distinguished from unwanted or non-specific interactions. The ability of an antibody to bind to a specific antigenic determinant can be measured through the enzyme-linked immunosorbent assay (ELISA) or other techniques familiar to those skilled in the art.

When the variable regions in the present disclosure are used to prepare an antibody, a binding molecule, a bispecific binding molecule, or a multispecific binding molecule, the constant region is not particularly limited. A constant region known to those skilled in the art or a self-obtained constant region may be used, and amino acid mutations (e.g., mutations that improve or reduce binding to an Fc receptor or FcRn) may also be introduced into the constant region moiety.

The method for obtaining the binding molecule, the antigen-binding fragment, the antibody, the bispecific binding molecule, or the multispecific binding molecule of the present disclosure is not particularly limited, and they can be obtained by any method. The binding molecule, the antigen-binding fragment, the antibody, the bispecific binding molecule, or the multispecific binding molecule of the present disclosure can be prepared and purified by conventional methods. For example, cDNA sequences encoding heavy and light chains can be cloned and recombined into an expression vector. CHO cells can be stably transfected with recombinant immunoglobulin expression vectors. As a more recommended prior art, mammalian expression systems will result in glycosylation of antibodies, particularly at the highly conserved N-terminus of the Fc region. Stable clones are obtained by expression of antibodies specifically binding to human antigens. Positive clones are expanded in a serum-free culture medium in a bioreactor to produce antibodies. The culture solution with the secreted antibodies can be purified and collected by conventional techniques. The antibodies can be filtered and concentrated by conventional methods. Soluble mixtures and polymers can also be removed by conventional methods, such as molecular sieves and ion exchange.

As used herein, the term "transfection" refers to the introduction of an exogenous nucleic acid into a eukaryotic cell. Transfection may be achieved by various means known in the art, including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

The term "stable transfection" refers to the introduction and integration of an exogenous nucleic acid, DNA, or RNA into the genome of a transfected cell. The term "stable transfectant" refers to a cell in which foreign DNA is stably integrated into the genomic DNA.

The term "antibody-drug conjugate" (ADC) refers to an antibody that has been covalently conjugated to a therapeutically active substance or active pharmaceutical ingredient (API), such that the therapeutically active substance or active pharmaceutical ingredient (API) can be directed to the binding target of the antibody, thereby exerting its pharmacological function. The therapeutically active substance or active pharmaceutical ingredient may be a cytotoxin that is capable of killing cells targeted by the ADC, preferably malignant or cancer cells. Covalent linkage of the therapeutically active substance, the active pharmaceutical ingredient, or the cytotoxin can be done in a non-site-specific manner using a standard chemical linker that conjugates the payload to a lysine or cysteine residue, or preferably, the conjugation is done in a site-specific manner that allows full control of the conjugation site and the drug-to-antibody ratio of the resulting ADC.

The term "amino acid substitution" or "substitution" means the replacement of an amino acid residue at a specific position in a parent polypeptide sequence with another amino acid residue.

The term "affinity" or "binding affinity" refers to the strength of the sum of all non-covalent interactions between a single binding site of a molecule (e.g., an antibody) and its binding ligand (e.g., an antigen). The term "$K_d$" refers to the dissociation constant of a particular antibody-antigen interaction. The binding affinity can be determined using various techniques known in the art, such as surface plasmon resonance, bio-layer interferometry, dual-polarization interferometry, static light scattering, dynamic light scattering, isothermal titration calorimetry, ELISA, analytical ultracentrifugation, flow cytometry, and the like.

The term "biological activity" refers to the ability of an antibody to bind to an antigen and cause a measurable biological response, which can be measured *in vitro* or *in vivo*.

The term "pharmaceutical composition" refers to a formulation or a combination of formulations containing one, two, or more active ingredients, which allows the active ingredients contained therein to be present in an effective biologically active form and does not contain additional ingredients having toxicity unacceptable to a subject to which the formulation is administered. The "pharmaceutical composition", when present in the form of a combination of separate formulations containing two or more different active ingredients, can be administered simultaneously, sequentially, separately, or intermittently, with the purpose of exerting the biological activity of the multiple active ingredients for jointly treating a disease.

The binding molecule or the antigen-binding fragment of the present disclosure can be used in combination with an additional drug. The active ingredients can be mixed together to form a single administration unit, or can be made into separate administration units for separate use.

The term "effective amount" refers to a dosage of the pharmaceutical formulation of the antibody or fragment of the present disclosure, which generates expected effects in a treated patient after the administration to the patient in a single or multiple doses. The effective amount can be easily determined by an attending physician as a person skilled in the art by considering a variety of factors as follows: such as ethnic differences; weight, age, and health status; the specific disease involved; the severity of the disease; response in an individual patient; specific antibody administered; mode of administration; bioavailability characteristics of the administered formulation; selected administration regimen; and use of any concomitant therapy.

As used herein, the term "individual" or "subject" refers to any animal, such as a mammal or a marsupial. The individual of the present disclosure includes, but is not limited to, humans, non-human primates (such as cynomolgus monkey or rhesus monkey or other types of macaques), mice, pigs, horses, donkeys, cows, sheep, rats, and any species of poultry.

As used herein, the term "disease", "condition", "disorder", or the like refers to any change or dysregulation that impairs or interferes with the normal function of cells, tissues, or organs. For example, the "disease" includes, but is not limited to: tumors, pathogen infections, autoimmune diseases, T cell dysfunctional diseases, or defects in immune tolerance (such as transplant rejection).

As used herein, the term "tumor" refers to a disease characterized by cells or tissues that undergo pathological proliferation and subsequently migrate to or invade other tissues or organs. Tumor growth is generally uncontrolled and progressive, without inducing or inhibiting normal cell proliferation.

As used herein, the term "treatment" refers to clinical intervention in an attempt to alter a disease process of an individual or to treat a cell-caused disease process, either prophylactically or during a clinical pathology. Therapeutic effects include, but are not limited to, preventing the occurrence or recurrence of a disease, alleviating a symptom, reducing any direct or indirect pathological consequences of a disease, preventing metastasis, slowing the progression of a disease, improving or relieving a condition, relieving or improving prognosis, etc.

The terms "G-protein coupled receptor family C group 5 member D" and "GPRC5D" particularly include human GPRC5D proteins, and include variants, subtypes, homologous species, and analogs having at least one common epitope with GPRC5D (e.g., human GPRC5D) of human GPRC5D. Exemplary human GPRC5D sequences can be those described in GenBank accession No. BC069341, NCBI reference sequence: NP_061124.1, and UniProtKB/Swiss-Prot accession No. Q9NZD1 (see also Brauner-Osborne, H et al., 2001, Biochim. Biophys. Acta 1518, 237-248).

DETAILED DESCRIPTION

The present disclosure is further illustrated with reference to the following specific examples. It should be understood that these examples are merely intended to illustrate the present disclosure rather than limit the scope of the present disclosure. Procedures without specified conditions in the following examples are generally conducted in conventional conditions, or conditions recommended by the manufacturers.

### Example 1. Design and Sequences of Anti-CD3-GPRC5D Bispecific Antibodies

The bispecific antigen-binding molecules constructed in this example (hereinafter referred to as bispecific antibodies) were formed by linking the heavy chain of the anti-GPRC5D full-length antibody Fab fragment to the binding domain of the human T cell receptor subunit CD3ε via a flexible linker. The anti-GPRC5D full-length antibody was a GPRC5D mAb, with its heavy chain sequence set forth in SEQ ID NO: 5 and its light chain sequence set forth in SEQ ID NO: 3. The CD3ε binding domain was derived from the full-length antibody CD3 mAb, with its heavy chain sequence set forth in SEQ ID NO: 6 and its light chain sequence set forth in SEQ ID NO: 7. To reduce the ADCC activity of the antibodies, amino acid substitutions L234A and L235A had been applied to the Fc fragments of the finally constructed bispecific antibodies.

The heavy chain variable region and the light chain variable region of the CD3 mAb were linked via a flexible linker to form a single-chain variable fragment (scFv) with a structure of VH-$(G_4S)_3$-VL and the sequence set forth in SEQ ID NO: 8. The scFv was then fused to the C-terminus of the heavy chain of the anti-GPRC5D full-length antibody Fab fragment via a flexible linker.

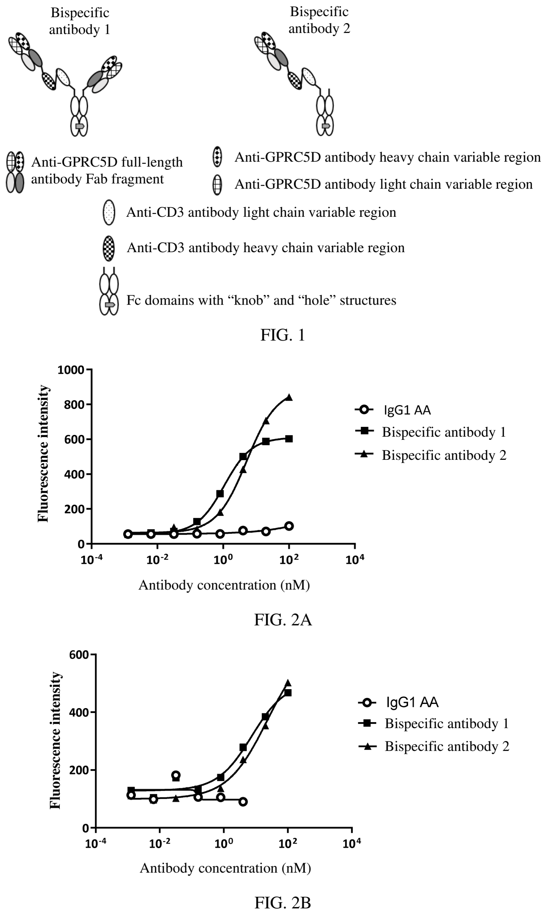

The scFv was fused to the C-terminus of the heavy chain of the anti-GPRC5D full-length antibody Fab fragment and simultaneously comprised another intact GPRC5D-binding domain and an Fc moiety with "knob" and "hole" structures, and the formed bispecific antibody was designated as bispecific antibody 1. The sequences of the fused chains are set forth in SEQ ID NO: 1, SEQ ID NO: 2, and SEQ ID NO: 3, and the schematic diagram is shown in FIG. 1.

The scFv was fused to the C-terminus of the heavy chain of the anti-GPRC5D full-length antibody Fab fragment and simultaneously comprised an Fc moiety with "knob" and "hole" structures, and the formed bispecific antibody was designated as bispecific antibody 2. The sequences of the fused chains are set forth in SEQ ID NO: 1, SEQ ID NO: 3, and SEQ ID NO: 4, and the schematic diagram is shown in FIG. 1.

Of the two heterologous heavy chains, the heavy chain containing the scFv was designed as a "knob" structure, which comprised amino acid substitutions at two sites: S354C and T366W. Of the two heterologous heavy chains, the heavy chain not containing the scFv was designed as a "hole" structure, which comprised amino acid substitutions at four sites: Y349C, T366S, L368A, and Y407V. In addition, to facilitate the purification of the bispecific antibodies, the heavy chain with the "hole" structure also underwent an H435R substitution.

The structures and related molecular sequences of bispecific antibody 1 and bispecific antibody 2 are summarized in Table 1 and Table 2, respectively.

Table 1. Structures of bispecific antibodies

| Molecular name | Molecular structure description | SEQ ID NO: |
|---|---|---|
| Bispecific antibody 1 | GPRC5D VH - GPRC5D CH1 - $G_4S$ - CD3VH - $(G_4S)_3$ - CD3VL - (EPKSS) - IgG1 Fc(knob) | 1 |
| | GPRC5D VH - GPRC5D CH1 - IgG1 Fc (hole) | 2 |
| | GPRC5D VL-GPRC5D CL (i.e., GPRC5D LC) $\times$ 2 | 3 |
| Bispecific antibody 2 | GPRC5D VH - GPRC5D CH1 - $G_4S$ - CD3VH - $(G_4S)_3$ - CD3VL - (EPKSS) - IgG1 Fc(knob) | 1 |
| | GPRC5D VL-GPRC5D CL (i.e., GPRC5D LC) | 3 |
| | IgG1 Fc(hole) | 4 |
| GPRC5D mAb | GPRC5D HC | 5 |
| | GPRC5D LC | 3 |
| CD3 mAb | CD3 HC | 6 |
| | CD3 LC | 7 |
| CD3 scFv | CD3VH - $(G_4S)_3$ - CD3VL | 8 |

Table 2. Amino acid sequences of bispecific antibodies

| Name | SEQ ID NO: | Amino acid sequence |
|------|------------|---------------------|
| "Knob" structure heavy chain of bispecific antibody 1 (GPRC5D VH-GPRC5D CH1-G$_4$S-CD3VH-(G$_4$S)$_3$-CD3VL-(EPKSS)-IgG1 Fc (knob)) | 1 | QVQLVQSGAEVKKPGASVKVSCKASGYTFTSYIIHWVRQA PGQGLEWIGYINPYNDGTKYNEKFKGRVTMTSDTSTSTVY MELSSLRSEDTAVYYCARGGIRAFMNYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSCGGGGSEVKLVESGGGLVQPGG SLKLSCAASGFTFNTYAMNWVRQAPGKGLEWVARIRSKY NNYATYYADSVKDRFTISRDDAKNTLYLQMNNLRTEDTAV YYCVRHGNFGNSYVSWFAYWGQGTLVTVSSGGGGSGGG GSGGGGSQAVVTQEPSLTVSPGGTVTLTCRSSTGAVTTSNY ANWVQQKPGQAPRGLIGGTNKRAPGTPARFSGSLLGGKA ALTLSGAQPEDEAEYYCALWYSNLWVFGGGTKLTVLEPKS SDKTHTCPPCPAPEAAGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISKA KGQPREPQVYTLPPCRDELTKNQVSLWCLVKGFYPSDIAVE WESNGQPENNYKTTPPVLDSDGSFFLYSKLTVDKSRWQQG NVFSCSVMHEALHNHYTQKSLSLSPGK |
| "Hole" structure heavy chain of bispecific antibody 1 (GPRC5D VH-GPRC5D CH1-IgG1 Fc (hole)) | 2 | QVQLVQSGAEVKKPGASVKVSCKASGYTFTSYIIHWVRQA PGQGLEWIGYINPYNDGTKYNEKFKGRVTMTSDTSTSTVY MELSSLRSEDTAVYYCARGGIRAFMNYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSCDKTHTCPPCPAPEAAGGPSVFL FPPKPKDTLMISRTPEVTCVVVDVSHEDPEVKFNWYVDGV EVHNAKTKPREEQYNSTYRVVSVLTVLHQDWLNGKEYKC KVSNKALPAPIEKTISKAKGQPREPQVCTLPPSRDELTKNQ VSLSCAVKGFYPSDIAVEWESNGQPENNYKTTPPVLDSDGS FFLVSKLTVDKSRWQQGNVFSCSVMHEALHNRYTQKSLSL SPGK |

| Name | SEQ ID NO: | Amino acid sequence |
|---|---|---|
| Light chain of bispecific antibody 1 (GPRC5D VL-GPRC5D CL) | 3 | DIQMTQSPSSLSASVGDRVTITCKASQDVGTAVAWYQQKP GKAPKLLIYWASTRHTGVPSRFSGSGSGTDFTLTISSLQPED FATYYCQHYISWTFGQGTKVEIKRTVAAPSVFIFPPSDEQLK SGTASVVCLLNNFYPREAKVQWKVDNALQSGNSQESVTE QDSKDSTYSLSSTLTLSKADYEKHKVYACEVTHQGLSSPV TKSFNRGEC |
| "Knob" structure heavy chain of bispecific antibody 2 (GPRC5D VH-GPRC5D CH1-G$_4$S-CD3VH-(G$_4$S)$_3$-CD3VL-(EPKSS)-IgG1 Fc (knob)) | 1 | QVQLVQSGAEVKKPGASVKVSCKASGYTFTSYIIHWVRQA PGQGLEWIGYINPYNDGTKYNEKFKGRVTMTSDTSTSTVY MELSSLRSEDTAVYYCARGGIRAFMNYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSCGGGGSEVKLVESGGGLVQPGG SLKLSCAASGFTFNTYAMNWVRQAPGKGLEWVARIRSKY NNYATYYADSVKDRFTISRDDAKNTLYLQMNNLRTEDTAV YYCVRHGNFGNSYVSWFAYWGQGTLVTVSSGGGGSGGG GSGGGGSQAVVTQEPSLTVSPGGTVTLTCRSSTGAVTTSNY ANWVQQKPGQAPRGLIGGTNKRAPGTPARFSGSLLGGKA ALTLSGAQPEDEAEYYCALWYSNLWVFGGGTKLTVLEPKS SDKTHTCPPCPAPEAAGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISKA KGQPREPQVYTLPPCRDELTKNQVSLWCLVKGFYPSDIAVE WESNGQPENNYKTTPPVLDSDGSFFLYSKLTVDKSRWQQG NVFSCSVMHEALHNHYTQKSLSLSPGK |
| "Hole" structure heavy chain of bispecific antibody 2 (IgG1 Fc (hole)) | 4 | DKTHTCPPCPAPEAAGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKAK GQPREPQVCTLPPSRDELTKNQVSLSCAVKGFYPSDIAVEW ESNGQPENNYKTTPPVLDSDGSFFLVSKLTVDKSRWQQGN VFSCSVMHEALHNRYTQKSLSLSPGK |

(continued)

| Name | SEQ ID NO: | Amino acid sequence |
|---|---|---|
| Light chain of bispecific antibody 2 (GPRC5D VL-GPRC5D CL) | 3 | DIQMTQSPSSLSASVGDRVTITCKASQDVGTAVAWYQQKP GKAPKLLIYWASTRHTGVPSRFSGSGSGTDFTLTISSLQPED FATYYCQHYISWTFGQGTKVEIKRTVAAPSVFIFPPSDEQLK SGTASVVCLLNNFYPREAKVQWKVDNALQSGNSQESVTE QDSKDSTYSLSSTLTLSKADYEKHKVYACEVTHQGLSSPV TKSFNRGEC |
| GPRC5D mAb heavy chain (GPRC5D HC) | 5 | QVQLVQSGAEVKKPGASVKVSCKASGYTFTSYIIHWVRQA PGQGLEWIGYINPYNDGTKYNEKFKGRVTMTSDTSTSTVY MELSSLRSEDTAVYYCARGGIRAFMNYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSCDKTHTCPPCPAPELLGGPSVFLF PPKPKDTLMISRTPEVTCVVVDVSHEDPEVKFNWYVDGVE VHNAKTKPREEQYNSTYRVVSVLTVLHQDWLNGKEYKCK VSNKALPAPIEKTISKAKGQPREPQVYTLPPSRDELTKNQVS LTCLVKGFYPSDIAVEWESNGQPENNYKTTPPVLDSDGSFF LYSKLTVDKSRWQQGNVFSCSVMHEALHNHYTQKSLSLSP GK |
| GPRC5D mAb light chain (GPRC5D LC) | 3 | DIQMTQSPSSLSASVGDRVTITCKASQDVGTAVAWYQQKP GKAPKLLIYWASTRHTGVPSRFSGSGSGTDFTLTISSLQPED FATYYCQHYISWTFGQGTKVEIKRTVAAPSVFIFPPSDEQLK SGTASVVCLLNNFYPREAKVQWKVDNALQSGNSQESVTE QDSKDSTYSLSSTLTLSKADYEKHKVYACEVTHQGLSSPV TKSFNRGEC |
| GPRC5D mAb heavy chain variable region VH | 9 | QVQLVQSGAEVKKPGASVKVSCKASGYTFTSYIIHWVRQA PGQGLEWIGYINPYNDGTKYNEKFKGRVTMTSDTSTSTVY MELSSLRSEDTAVYYCARGGIRAFMNYWGQGTLVTVSS |
| GPRC5D mAb light chain variable region VL | 10 | DIQMTQSPSSLSASVGDRVTITCKASQDVGTAVAWYQQKP GKAPKLLIYWASTRHTGVPSRFSGSGSGTDFTLTISSLQPED FATYYCQHYISWTFGQGTKVEIK |

| Name | SEQ ID NO: | Amino acid sequence |
|---|---|---|
| GPRC5D mAb heavy chain HCDR1 | 11 | SYIIH |
| GPRC5D mAb heavy chain HCDR2 | 12 | YINPYNDGTKYNEKFKG |
| GPRC5D mAb heavy chain HCDR3 | 13 | GGIRAFMNY |
| GPRC5D mAb light chain LCDR1 | 14 | KASQDVGTAVA |
| GPRC5D mAb light chain LCDR2 | 15 | WASTRHT |
| GPRC5D mAb light chain LCDR3 | 16 | QHYISWT |
| CD3 mAb heavy chain (CD3 HC) | 6 | EVKLVESGGGLVQPGGSLKLSCAASGFTFNTYAMNWVRQ APGKGLEWVARIRSKYNNYATYYADSVKDRFTISRDDAKN TLYLQMNNLRTEDTAVYYCVRHGNFGNSYVSWFAYWGQ GTLVTVSSASTKGPSVFPLAPSSKSTSGGTAALGCLVKDYF PEPVTVSWNSGALTSGVHTFPAVLQSSGLYSLSSVVTVPSSS LGTQTYICNVNHKPSNTKVDKKVEPKSCDKTHTCPPCPAP EAAGGPSVFLFPPKPKDTLMISRTPEVTCVVVDVSHEDPEV KFNWYVDGVEVHNAKTKPREEQYNSTYRVVSVLTVLHQD WLNGKEYKCKVSNKALPAPIEKTISKAKGQPREPQVYTLP PSRDELTKNQVSLTCLVKGFYPSDIAVEWESNGQPENNYKT TPPVLDSDGSFFLYSKLTVDKSRWQQGNVFSCSVMHEALH NHYTQKSLSLSPGK |
| CD3 mAb light chain (CD3 LC) | 7 | QAVVTQEPSLTVSPGGTVTLTCRSSTGAVTTSNYANWVQQ KPGQAPRGLIGGTNKRAPGTPARFSGSLLGGKAALTLSGA QPEDEAEYYCALWYSNLWVFGGGTKLTVLGQPKANPTVT LFPPSSEELQANKATLVCLISDFYPGAVTVAWKADSSPVKA GVETTTPSKQSNNKYAASSYLSLTPEQWKSHRSYSCQVTH EGSTVEKTVAPTEC |
| CD3 mAb heavy chain variable region VH | 17 | EVKLVESGGGLVQPGGSLKLSCAASGFTFNTYAMNWVRQ APGKGLEWVARIRSKYNNYATYYADSVKDRFTISRDDAKN TLYLQMNNLRTEDTAVYYCVRHGNFGNSYVSWFAYWGQ GTLVTVSS |

| Name | SEQ ID NO: | Amino acid sequence |
|---|---|---|
| CD3 mAb light chain variable region VL | 18 | QAVVTQEPSLTVSPGGTVTLTCRSSTGAVTTSNYANWVQQ KPGQAPRGLIGGTNKRAPGTPARFSGSLLGGKAALTLSGA QPEDEAEYYCALWYSNLWVFGGGTKLTVL |
| scFv of CD3 mAb | 8 | EVKLVESGGGLVQPGGSLKLSCAASGFTFNTYAMNWVRQ APGKGLEWVARIRSKYNNYATYYADSVKDRFTISRDDAKN TLYLQMNNLRTEDTAVYYCVRHGNFGNSYVSWFAYWGQ GTLVTVSSGGGGSGGGGSGGGGSQAVVTQEPSLTVSPGGT VTLTCRSSTGAVTTSNYANWVQQKPGQAPRGLIGGTNKRA PGTPARFSGSLLGGKAALTLSGAQPEDEAEYYCALWYSNL WVFGGGTKLTVL |
| CD3 mAb heavy chain HCDR1 | 19 | TYAMN |
| CD3 mAb heavy chain HCDR2 | 20 | RIRSKYNNYATYYADSVKD |
| CD3 mAb heavy chain HCDR3 | 21 | HGNFGNSYVSWFAY |
| CD3 mAb light chain LCDR1 | 22 | RSSTGAVTTSNYAN |
| CD3 mAb light chain LCDR2 | 23 | GTNKRAP |
| CD3 mAb light chain LCDR3 | 24 | ALWYSNLWV |
| Fab heavy chain of bispecific antibody 1 or bispecific antibody 2 (GPRC5D VH-GPRC5D CH1) | 25 | QVQLVQSGAEVKKPGASVKVSCKASGYTFTSYIIHWVRQA PGQGLEWIGYINPYNDGTKYNEKFKGRVTMTSDTSTSTVY MELSSLRSEDTAVYYCARGGIRAFMNYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSC |
| "Knob" structure heavy chain Fc domain of bispecific antibody 1 or bispecific antibody 2 (IgG1 Fc (knob)) | 26 | DKTHTCPPCPAPEAAGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKAK GQPREPQVYTLPPCRDELTKNQVSLWCLVKGFYPSDIAVE WESNGQPENNYKTTPPVLDSDGSFFLYSKLTVDKSRWQQG NVFSCSVMHEALHNHYTQKSLSLSPGK |

EP 4 714 974 A1

## Example 2. Construction of Anti-CD3-GPRC5D Bispecific Antibodies and Transient Transfection and Expression of Anti-CD3-GPRC5D Bispecific Antibodies in Eukaryotic Cells

The gene fragments encoding the bispecific antibody molecules described above were separately cloned into pTT5 expression vectors to prepare transfection-grade expression plasmids.

Expi293F™ cells (Thermo Fisher Scientific) were cultured in a serum-free culture medium, seeded in a shake flask (Corning Inc.), and cultured on a shaker at 37 °C with 8% $CO_2$. The cell density was adjusted, and the recombinant expression vector containing the target gene fragment and the PEI transfection reagent were mixed according to a suitable ratio and added to a cell culture shake flask. After 6 days of cell culture, the expression supernatant was collected, centrifuged at high speed to remove cell debris, and subjected to affinity purification using a Protein A column. The column was rinsed with PBS until the A280 reading dropped to the baseline. The target protein was eluted with acidic eluent of pH 3.0-pH 3.5, and neutralized with 1 M Tris-HCl (pH 8.0-9.0). The eluted sample was appropriately concentrated and then further purified by gel chromatography Superdex200 (GE) equilibrated with PBS to remove aggregates, and the monomer peak was collected, buffer-exchanged into PBS, and aliquoted for later use. The final purified antibodies were subjected to SDS-PAGE and HPLC for purity analysis and A280 concentration determination.

## Example 3. Affinity Assay on Anti-CD3-GPRC5D Bispecific Antibodies

A. Affinity assay of anti-CD3-GPRC5D bispecific antibodies for cells expressing hGPRC5D or hCD3

The binding of the anti-CD3-GPRC5D bispecific antibodies to NCI-H929 cells and RPMI8226 cells expressing hGPRC5D, as well as T lymphocytes naturally expressing hCD3 (Jurkat), was determined by FACS.

NCI-H929 cells (ATCC, CRL-9068), RPMI8226 cells (ATCC, CCL-155), and Jurkat cells (ATCC, TIB-152) were cultured. The culture medium for NCI-H929 cells was RPMI1640 + 10% FBS + 0.05 mM mercaptoethanol, and the culture medium for RPMI8226 and Jurkat cells was RPMI1640 + 10% FBS. The cells were cultured in a T75 cell culture flask in an incubator at 37 °C with 5% $CO_2$. When the cells were ready for use, NCI-H929 cells, RPMI8226 cells, and Jurkat cells were separately directly placed into a 50 mL centrifuge tube without digestion.

The resulting cells were centrifuged at 1000 rpm and room temperature for 5 min, and the supernatant was discarded. The cells were resuspended in 100 μL of 1% BSA (in PBS). The cells were counted, and the cell density was adjusted to 1E6 cells/mL. The cells were plated in a 96-well round-bottom culture plate (Corning 3799) and centrifuged at 1500 rpm and 4°C for 5 min, and the supernatant was discarded. The cells were resuspended in 200 μL of 1% BSA (in PBS) and centrifuged again at 1500 rpm and 4°C for 5 min, and the supernatant was discarded. The cells were left to stand at 4°C for later use. The antibodies to be tested and a negative control IgG1 AA (purchased from Biointron, Cat. No. B109802) were diluted with 1% BSA (in PBS) at an initial concentration of 100 nM and diluted in 10-fold dilution series to obtain 7 concentrations. The diluted antibody was added at 100 μL/well to resuspend the cells, and the mixture was incubated at 4°C for 1 h. The mixture was centrifuged at 1500 rpm and 4°C for 5 min, and the supernatant was discarded. The cells were resuspended in and washed with 160 μL of 1% BSA (in PBS) and centrifuged at 1500 rpm and 4°C for 5 min, and the supernatant was discarded. The secondary antibody (goat anti human IgG Fc PE) was diluted with 1% BSA (in PBS) at a ratio of 1:400 according to the instructions. The diluted secondary antibody was added at 100 μL/well to resuspend the cells, and the mixture was incubated at 4°C for 0.5 h. The mixture was centrifuged at 1500 rpm and 4°C for 5 min, and the supernatant was discarded. The cells were resuspended in and washed with 200 μL of 1% BSA (in PBS) and centrifuged at 1500 rpm and 4°C for 5 min, and the supernatant was discarded. The cells were resuspended in 100 μL of 1% BSA (in PBS) and filtered through 300-mesh gauze, and the mean fluorescence intensity in the PE channel was measured using a flow cytometer.

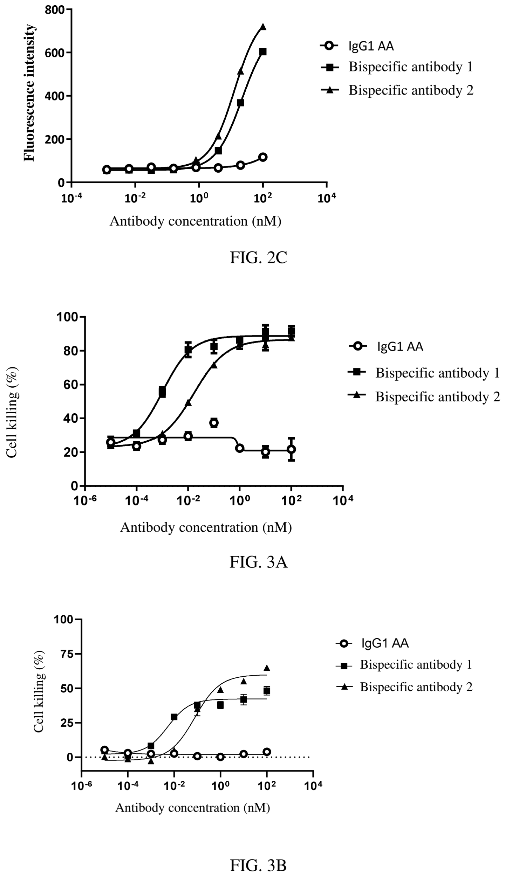

An FCS file was exported from the flow cytometer, and the PE channel mean fluorescence intensity (hereinafter referred to as MFI) of each sample was analyzed by using flowjo software. The analyzed mean fluorescence intensity was imported into Graphpad to analyze the half maximal binding concentration (hereinafter referred to as $EC_{50}$) and the top mean fluorescence intensity (Top MFI) of the antibodies to the cells. The results are shown in Table 3, FIG. 2A (NCI-H929 cells), FIG. 2B (RPMI8226 cells), and FIG. 2C (Jurkat cells). The binding ability of the two bispecific antibody molecules to the Jurkat cell strain was weaker than that of the two bispecific antibody molecules to the NCI-H929 cell strain. It was reported in the literature that the expression level of GPRC5D in RPMI8226 cells was lower than that in NCI-H929 cells. Therefore, the binding ability of the two bispecific antibody molecules to the RPMI8226 cell strain was weaker than that of the two bispecific antibody molecules to the NCI-H929 cell strain.

Table 3. Affinities of bispecific antibodies for hGPRC5D (NCI-H929 cell strain and RPMI8226 cell strain) and hCD3 (Jurkat cell strain)

| Molecule No. | NCI-H929 cell strain | | RPMI8226 cell strain | | Jurkat cell strain | |
|---|---|---|---|---|---|---|
| | $EC_{50}$ (nM) | Top | $EC_{50}$ (nM) | Top | $EC_{50}$ (nM) | Top |
| Bispecific antibody 1 | 1.07 | 603 | 7.48 | 467 | 22.14 | 604 |
| Bispecific antibody 2 | 5.19 | 842 | 22.86 | 502 | 12.87 | 720 |

*B. In vitro* recombinant protein binding affinity and kinetics of anti-CD3-GPRC5D bispecific antibodies

The affinity and kinetic properties of the anti-CD3-GPRC5D bispecific antibody molecules for human/cynomolgus monkey CD3 were analyzed using a Biacore 8K instrument.

To determine the affinity and kinetic properties for human CD3 (purchased from Acro, Cat. No. CDD-H52W1)/cynomolgus monkey CD3 (purchased from Acro, Cat. No. CDD-C52W4), a method for directly immobilizing human/cynomolgus monkey CD3 molecules on a CM5 chip was used. The CM5 chip was first activated with EDC and NHS. The human/cynomolgus monkey CD3 molecules were diluted to 1 μg/mL with an acetate solution (pH = 5), immobilized for 60 s at a flow rate of 10 μL/min, and then blocked with ethanolamine. The anti-CD3-GPRC5D bispecific antibody molecules were diluted in 2-fold dilution series to a series of concentrations (100 nM-0.39 nM) with an HBS-EP+ buffer (10 mM HEPES, pH 7.4, 150 mM NaCl, 3 mM EDTA, 0.05% P20) and injected at a flow rate of 50 μL/min for association for 90 s, followed by dissociation for 360 s.

After each round of the experiment was completed, the chip was regenerated by being rinsed with a 3 M MgCl$_2$ solution at a flow rate of 30 μL/min for 30 s to remove the anti-CD3-GPRC5D bispecific antibody molecules. The original data were analyzed using Biacore Insight Evaluation Software (3.0.12.15655) and fitted with a (1:1) Langmuir model. The obtained experimental data for the affinity and kinetics of the bispecific antibodies are shown in Table 4.

Table 4. Binding affinities and kinetics of anti-CD3-GPRC5D bispecific antibodies for human/cynomolgus monkey CD3 proteins

| Molecule No. | Antigen | Association rate $k_a$ (1/Ms) | Dissociation rate $k_d$ (1/s) | Affinity $K_D$ (M) |
|---|---|---|---|---|
| Bispecific antibody 1 | Human CD3 | 1.13E+05 | 2.26E-03 | 2.00E-08 |
| Bispecific antibody 2 | Human CD3 | 1.18E+05 | 2.14E-03 | 1.81E-08 |
| Bispecific antibody 1 | Cynomolgus monkey CD3 | 1.74E+05 | 2.92E-03 | 1.68E-08 |
| Bispecific antibody 2 | Cynomolgus monkey CD3 | 1.47E+05 | 2.79E-03 | 1.89E-08 |

The experimental results show that both bispecific antibody molecules bound to human/cynomolgus monkey CD3 with comparable affinities.

**Example 4. *In Vitro* Functional Experiment of Anti-CD3-GPRC5D Bispecific Antibodies**

A. T cell-dependent cell-mediated cytotoxicity (TDCC) experiment, with the target cells being NCI-H929

NCI-H929 cells (ATCC, CRL-9068) were cultured, and the culture medium was RPMI1640 + 10% FBS + 0.05 mM mercaptoethanol. The cells were cultured in a T75 cell culture flask in an incubator at 37 °C with 5% CO$_2$. When the cells were ready for use, NCI-H929 cells were directly placed into a 50 mL centrifuge tube without digestion. The cells were centrifuged at 1000 rpm for 5 min, and the supernatant was discarded. The cells were resuspended in an RPMI1640 + 2% FBS culture medium and counted, and the cell density was adjusted to 1.5E5 cells/mL.

The target cells were plated in a flat-bottom 96-well plate (Corning, 3599) at 100 μL/well and incubated overnight at 37°C with 5% CO$_2$. CD3+ T cells were sorted from fresh PBMCs using a T cell negative selection kit (StemCell, Cat. No. 17951), and the cells were counted. The cell density was adjusted to 3E6 cells/mL using RPMI1640 + 2% FBS. The effector cells were plated in the 96-well plate at 50 μL/well and cultured at 37°C with 5% CO$_2$.

The antibodies were diluted in 10-fold dilution series with an RPMI1640 + 2% FBS culture medium, with an initial concentration of 400 nM. The diluted antibodies were added to the cell culture plate at 50 μL/well, so that the initial final concentration was 100 nM. The plate was incubated at 37°C with 5% CO$_2$ for 24 h. The culture plate was centrifuged at 1000 rpm for 5 min. 50 μL of the supernatant was pipetted into another flat-bottom 96-well plate, and 50 μL of an LDH

detection reagent (Romos, 4744934001) was added to each well. The mixture was well mixed. The plate was centrifuged at 1000 rpm for 5 min and then incubated in the dark for 10 min. OD492 values were read on Envision.

The percentage of cell killing caused by the TDCC effect was calculated using the following formula:

Cell killing% = [(sample well - spontaneous release of T cells - spontaneous release of target cells)/(maximum lysis of target cells - spontaneous release of target cells)] $\times$ 100%.

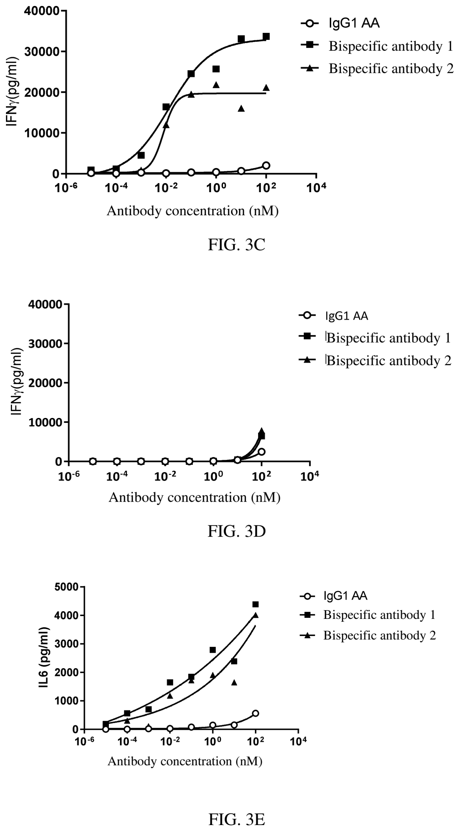

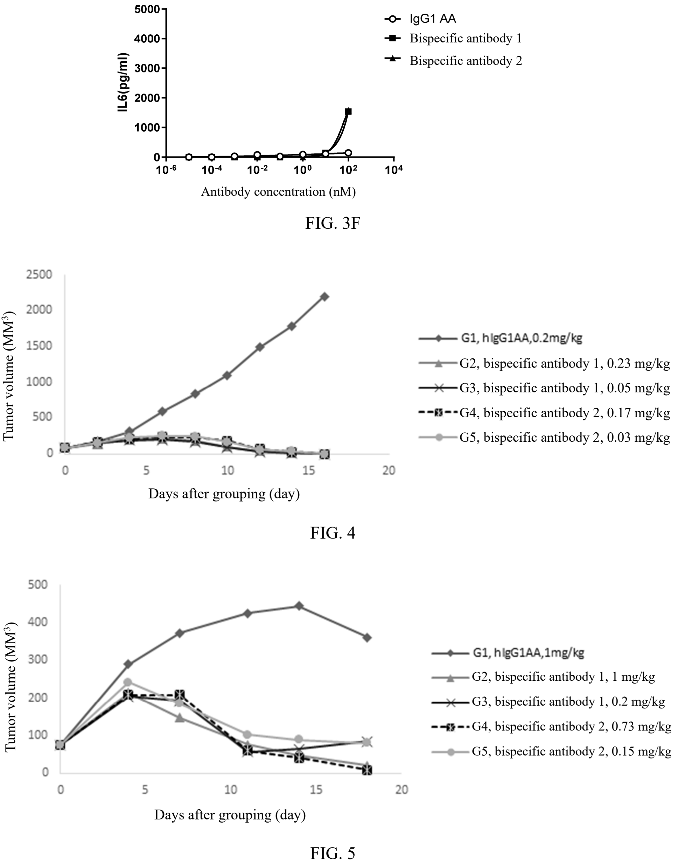

The cell killing data were imported into GraphPad Prism. The cell killing/concentration curves were plotted, and the $EC_{50}$ values were calculated. The results are shown in Table 5 and FIG. 3A. The maximum killing ability of the two bispecific antibody molecules to NCI-H929 cells could reach about 90%, and the median effective concentration of the bispecific antibody 1 molecule was lower than that of the bispecific antibody 2 molecule.

B. T cell-dependent cell-mediated cytotoxicity (TDCC) experiment, with the target cells being RPMI8226

RPMI8226 cells (ATCC, CCL-155) were cultured, and the culture medium was RPMI1640 + 10% FBS. The cells were cultured in a T75 cell culture flask in an incubator at 37°C with 5% $CO_2$. When the cells were ready for use, RPMI8226 cells were directly placed into a 50 mL centrifuge tube without digestion. The cells were centrifuged at 1000 rpm for 5 min, and the supernatant was discarded. The cells were resuspended in an RPMI1640 + 2% FBS culture medium and counted, and the cell density was adjusted to 1.5E5 cells/mL.

The target cells were plated in a flat-bottom 96-well plate (Corning, 3599) at 100 $\mu$L/well and incubated overnight at 37°C with 5% $CO_2$. CD3+ T cells were sorted from fresh PBMCs using a T cell negative selection kit (StemCell, Cat. No. 17951), and the cells were counted. The cell density was adjusted to 3E6 cells/mL using RPMI1640 + 2% FBS. The effector cells were plated in the 96-well plate at 50 $\mu$L/well and cultured at 37 °C with 5% $CO_2$.

The antibodies were diluted in 10-fold dilution series with an RPMI1640 + 2% FBS culture medium, with an initial concentration of 400 nM. The diluted antibodies were added to the cell culture plate at 50 $\mu$L/well, so that the initial final concentration was 100 nM. The plate was incubated at 37°C with 5% $CO_2$ for 24 h. The culture plate was centrifuged at 1000 rpm for 5 min. 50 $\mu$L of the supernatant was pipetted into another flat-bottom 96-well plate, and 50 $\mu$L of an LDH detection reagent (Romos, 4744934001) was added to each well. The mixture was well mixed. The plate was centrifuged at 1000 rpm for 5 min and then incubated in the dark for 10 min. OD492 values were read on Envision.

The percentage of cell killing caused by the TDCC effect was calculated using the following formula:

Cell killing% = [(sample well - spontaneous release of T cells - spontaneous release of target cells)/(maximum lysis of target cells - spontaneous release of target cells)] $\times$ 100%.

The cell killing data were imported into GraphPad Prism. The cell killing/concentration curves were plotted, and the $EC_{50}$ values were calculated. The results are shown in Table 5 and FIG. 3B. It was reported in the literature that the expression level of GPRC5D in RPMI8226 cells was lower than that in NCI-H929 cells. Therefore, the maximum killing ability of the two bispecific antibody molecules to RPMI8226 cells was far weaker than that of the two bispecific antibody molecules to NCI-H929 cells; the median effective concentration of the two bispecific antibody molecules for killing RPMI8226 cells was higher than that of the two bispecific antibody molecules for killing NCI-H929 cells; the killing ability of the bispecific antibody 1 molecule was stronger than that of the bispecific antibody 2 molecule.

Table 5. Results of T cell-dependent cell-mediated cytotoxicity (TDCC) experiment of anti-CD3-GPRC5D bispecific antibodies

| Molecule No. | NCI-H929 cell strain | | RPMI8226 cell strain | |
|---|---|---|---|---|
| | $EC_{50}$(nM) | Maximum killing (%) | $EC_{50}$(nM) | Maximum killing (%) |
| Bispecific antibody 1 | 0.001 | 92 | 0.01 | 42 |
| Bispecific antibody 2 | 0.017 | 88 | 0.08 | 60 |

C. Cytokine release experiment

NCI-H929 cells (ATCC, CRL-9068) were cultured, and the culture medium was RPMI1640 + 10% FBS + 0.05 mM mercaptoethanol. The cells were cultured in a T75 cell culture flask in an incubator at 37°C with 5% $CO_2$. When the cells were ready for use, NCI-H929 cells were directly placed into a 50 mL centrifuge tube without digestion. The cells were centrifuged at 1000 rpm for 5 min, and the supernatant was discarded. The cells were resuspended in an RPMI1640 + 2% FBS culture medium and counted, and the cell density was adjusted to 1.5E5 cells/mL. NCI-H929 cells were plated in a 96-well plate (Corning 3799) at 50 $\mu$L/well and cultured at 37°C with 5% $CO_2$.

Fresh PBMCs were purchased and counted, and the cell density was adjusted to 6E6 cells/mL with RPMI1640 + 2% FBS. The effector cells were plated in the 96-well plate at 50 $\mu$L/well and cultured at 37°C with 5% $CO_2$.

The antibodies were diluted in 10-fold dilution series with an RPMI1640 + 2% FBS culture medium, with an initial concentration of 400 nM. The diluted antibodies were added to the cell culture plate at 50 $\mu$L/well, so that the initial final concentration was 100 nM. The plate was incubated at 37°C with 5% $CO_2$ for 24 h.

The culture plate was centrifuged at 400 g for 10 min, and 100 $\mu$L of the supernatant was taken and cryopreserved at -80 °C for later use.

The standard was diluted in 2-fold dilution series according to the instructions of the cytokine detection kit (Biolegend, 430104 (Human IFN-$\gamma$ ELISA MAX Deluxe); 430504 (ELISA MAX Deluxe Set Human IL-6)) to obtain a maximum concentration of 8000 pg/mL and a minimum concentration of 0 pg/mL, with 12 concentration points in total. The prepared standard or thawed sample was added to a 96-well plate at 100 $\mu$L/well (the samples all required additional dilution), and the OD450 values were read on Envision according to the kit operation method.

The results are shown in FIGs. 3C-3F. In the case of co-incubation with PBMCs and tumor cells, the release levels of IFN$\gamma$ and IL6 cytokines induced by the bispecific antibody 2 molecule were all lower than those induced by the bispecific antibody 1 molecule. Under the PBMC-only condition, the release levels of cytokines of both bispecific antibody molecules were relatively low. Under the condition of co-incubation with PBMCs and NCI-H929 tumor cells, the bispecific antibody molecules had a suitable safety window.

## Example 5. Physical Stability Test of Anti-CD3-GPRC5D Bispecific Antibodies

The thermal stability of different antibodies in PBS buffer at pH 7.4 was detected by NanoDSF (differential scanning fluorimetry technique). The sample concentration was about 1 mg/mL, and detection was performed using Prometheus NT.Plex (nano DSF). Before the detection, each sample was centrifuged at 10000 g for 10 min. 40 $\mu$L of the sample was added to each well of the sample plate (the sample loading amount of the instrument was 10 $\mu$L, and each sample had one replicate well). The scanning started at a temperature of 30°C and ended at a temperature of 95°C, with a scanning rate of 0.5°C/min. The experimental results are shown in Table 6. Both bispecific antibody molecules showed good thermal stability.

Table 6. NanoDSF detection results of anti-CD3-GPRC5D bispecific antibodies

| Molecule No. | Tm Onset | Tm1 | Tagg |
|---|---|---|---|
| Bispecific antibody 1 | 58.4 °C | 61.6 °C | 78.6 °C |
| Bispecific antibody 2 | 57.1 °C | 61.8 °C | 77.8 °C |

## Example 6. Pharmacokinetic Experiment of Anti-CD3-GPRC5D Bispecific Antibodies

Two naive cynomolgus monkeys were used in the experiment, with free access to water. The administration dose of the bispecific antibodies was 1 mg/kg, and the intravenous infusion was completed within 30 min. The blood collection time points were Pre-dose, 30 min, 2 hr, 4 hr, 6 hr, 24 hr (1 d), 48 hr (2 d), 72 hr (3 d), 120 hr (5 d), 168 hr (7 d), 240 hr (10 d), and 336 hr (14 d). The whole blood samples were collected in an anticoagulant-free polyethylene tube, left to stand at room temperature for about 1 h, centrifuged at 6000 g and 25°C, and immediately aliquoted into two parts (PK samples and cytokine detection samples), which were immediately placed on dry ice and transferred to a refrigerator at -80 °C for long-term storage.

The concentration of the CD3 moiety in the serum was determined by ELISA. A 96-well plate was coated with a human CD3 protein with a concentration of 1 $\mu$g/mL at 100 $\mu$L/well and left to stand overnight at 4 °C. The plate was washed 3 times with PBST at 200 $\mu$L/well. 300 $\mu$L of a blocking reagent (5% milk powder) was added, and the plate was incubated at 37°C for 1 h. The plate was washed 3 times with PBST at 200 $\mu$L/well. 100 $\mu$L of the sample to be tested was added, and the plate was incubated at 37°C for 1 h. The plate was washed 6 times with PBST at 300 $\mu$L/well, and anti-human IgG (H&L) HRP (1:2000) and TMB were then added. After the plate was left to stand in the dark for 10 min, 100 $\mu$L of a stop solution was added to stop the color development reaction. The concentration of the CD3 moiety was quantified based on the color reaction.

The absorbance values at a wavelength of 450 nm were measured using an M5 plate reader from MD, and the data were processed using softmax software.

The monkey serum concentrations of the bispecific antibodies were calculated using Phoenix Winnolin 8.2 software to obtain the pharmacokinetic parameters for the bispecific antibodies.

**EP 4 714 974 A1**

The results show that the pharmacokinetic properties of bispecific antibody 1 and bispecific antibody 2 were good.

**Example 7. Pharmacokinetic Experiment of Anti-CD3-GPRC5D Bispecific Antibodies with Cytokine Detection**

The detection kit was a Muti-Analyte Flow assay kit (Biolegend, Cat. No. 740391). Before use, 250 $\mu$L of a buffer was added to lyophilized NHP Th cytokines, and the mixture was well mixed and left to stand at room temperature for 10 min. The standard was diluted in a ratio of 1:4 with the assay buffer in the kit, and the PK serum sample was diluted in a ratio of 1:4 with the assay buffer in the kit. 10 mL of LEGENDplex Assay Buffer was added to the lyophilized Matrix B for dissolution at room temperature for 15 min for later use.

25 $\mu$L of Matrix B and 25 $\mu$L of the standard were added to the standard wells, and 25 $\mu$L of an assay buffer and 25 $\mu$L of the serum sample were added to the sample wells. The detection beads were well mixed by vortex, and then the various detection beads were mixed at a ratio of 1:1 and diluted to a working concentration with an assay buffer, with 25 $\mu$L added to each well. The plate was sealed with a sealing film, shaken at 800 rpm, and incubated in the dark for 2 h. The plate was centrifuged at 250 g for 5 min, and the supernatant was discarded. The plate was washed with a wash buffer at 200 $\mu$L/well. 25 $\mu$L of a detection antibody was added to the plate, and the plate was sealed with a sealing film, shaken at 600 rpm and room temperature, and incubated in the dark for 1 h. 25 $\mu$L of SA-PE was added, and the plate was sealed with a sealing film, shaken at 600 rpm and room temperature, and incubated in the dark for 0.5 h. The plate was centrifuged at 250 g and room temperature for 5 min, and the supernatant was discarded. The plate was washed with a wash buffer at 200 $\mu$L/well. The sample was resuspended in 1% BSA (in PBS), filtered through 300-mesh gauze, and detected by flow cytometry.

An FCS file was exported, and the cytokine release levels of the samples were analyzed using LENEGDplex 8.0 software. The results are shown in Table 7.

Table 7. Results of pharmacokinetic experiment of anti-CD3-GPRC5D bispecific antibodies with cytokine detection

| | | Sampling time point | IL-2 (pg/mL) | IL-4 (pg/mL) | IL-5 (pg/mL) | IL-6 (pg/mL) | TNF$\alpha$ (pg/mL) | IFN$\gamma$ (pg/mL) |
|---|---|---|---|---|---|---|---|---|
| Bispecific antibody 1 | Male cynomolgus monkey | 0 h | 8.52 | 3.1 | 1.67 | 5.42 | 7.58 | 3.14 |
| | | 2 h | 8.61 | 2.1 | 1.64 | 13.48 | 4.3 | 2.62 |
| | | 6 h | 9.07 | 1.84 | 1.99 | 49.07 | 4.5 | 14.47 |
| | | 24 h | 8.59 | 1.85 | 2.19 | 126.11 | 5.33 | 2.66 |
| | | 48 h | 8.49 | 1.88 | 1.79 | 60.64 | 3.95 | 2.67 |
| | | 72 h | 6.83 | 4.79 | 2.72 | 5.53 | 4.01 | 2.64 |
| | Female cynomolgus monkey | 0 h | 6.83 | 4.7 | 1.84 | 5.08 | 4.52 | 4.24 |
| | | 2 h | 6.83 | 2.7 | 1.62 | 11.45 | 4.56 | 2.8 |
| | | 6 h | 6.8 | 1.85 | 1.47 | 27.96 | 4.84 | 3.33 |
| | | 24 h | 6.81 | 1.82 | 1.5 | 15.61 | 4.72 | 3.16 |
| | | 48 h | 6.78 | 1.8 | 1.41 | 36.16 | 4.66 | 2.91 |
| | | 72 h | 6.81 | 1.8 | 1.36 | 12.91 | 4.6 | 2.95 |

(continued)

|  |  | Sampling time point | IL-2 (pg/mL) | IL-4 (pg/mL) | IL-5 (pg/mL) | IL-6 (pg/mL) | TNFα (pg/mL) | IFNγ (pg/mL) |
|---|---|---|---|---|---|---|---|---|
| Bispecific antibody 2 | Male cynomolgus monkey | 0 h | 6.77 | 1.79 | 4.85 | 16.28 | 5.06 | 6.02 |
|  |  | 2 h | 6.81 | 3.97 | 1.56 | 35.37 | 4.88 | 2.95 |
|  |  | 6 h | 6.85 | 1.77 | 1.62 | 35.6 | 4.88 | 3.09 |
|  |  | 24 h | 6.84 | 6.84 | 1.92 | 306.69 | 4.28 | 3 |
|  |  | 48 h | 6.84 | 1.87 | 1.59 | 16.17 | 4.34 | 2.92 |
|  |  | 72 h | 6.84 | 1.86 | 1.42 | 8.23 | 4.17 | 30.38 |
|  | Female cynomolgus monkey | 0 h | 6.79 | 1.75 | 4.34 | 12.91 | 4.15 | 3.28 |
|  |  | 2 h | 6.78 | 1.84 | 2.14 | 29.65 | 5.81 | 3.56 |
|  |  | 6 h | 6.8 | 1.89 | 5.56 | 61.65 | 6.04 | 3.06 |
|  |  | 24 h | 6.78 | 1.76 | 2.72 | 744.54 | 5.49 | 3.06 |
|  |  | 48 h | 6.8 | 7.58 | 2.16 | 31.33 | 4.62 | 3 |
|  |  | 72 h | 6.8 | 1.8 | 1.74 | 9.62 | 4.25 | 2.6 |

The experimental results show that during the pharmacokinetic experiment on cynomolgus monkeys, both bispecific antibodies were detected for cytokine release. The overall levels of cytokine release were very low. The release level of IL6 was slightly high, but it returned to the initial level after 72 h. This indicates that the antibodies have good safety profiles.

## Example 8. *In Vivo* Efficacy Experiment of Anti-CD3-GPRC5D Bispecific Antibodies

A. Human immune cell reconstitution mouse model, with tumor cells being NCI-H929

The experimental animals were all housed in separate ventilated boxes with constant temperature and humidity. The feeding room was maintained at a temperature of 20.0-26.0°C, with humidity of 40-70% and a light/dark cycle of 12 h/12 h.

NCI-H929 cells were cultured in an RPMI 1640 culture medium containing 10% fetal bovine serum and 0.05 mM β-ME. NCI-H929 cells in the exponential growth phase were collected and resuspended in 0.1 mL of suspension of PBS and matrigel (1:1), and $5 \times 10^6$ NCI-H929 cells were inoculated into the upper right dorsum of the experimental mice. The growth of the tumors was observed periodically. When the mean volume of the tumors reached 79 mm$^3$, the mice were randomly grouped for administration according to the tumor size and the mouse body weight.

Donor PBMCs were purchased from AllCells, LLC. On the day of NCI H929 cell inoculation, each mouse was intraperitoneally injected with $1 \times 10^7$ PBMCs/0.1 mL PBS to establish a human immune cell reconstitution mouse model.

Before the start of administration, all the animals were weighed, and the tumor volume was measured with a vernier caliper. Given that tumor volume affects the effectiveness of treatment, the mice were grouped using the random grouping design based on their tumor volumes to ensure similar tumor volumes across different groups. Grouping was performed using StudyDirectorTM (version 3.1.399.19, supplier: Studylog System, Inc., S. San Francisco, CA, USA). The day of mouse grouping was recorded as day 0, and intraperitoneal injection was performed once every three days for 6 times starting from D0. The experimental groups and administration regimens for the hIgG1 AA negative control group (purchased from Biointron, Cat. No. B109802) and the test bispecific antibody molecules are shown in Table 8.

Table 8. Experimental groups and administration regimens

| Group No. | Number of mice per group | Test drug | Dose (mg/kg) | Route of administration | Actual administration frequency and period |
|---|---|---|---|---|---|
| G1 | 6 | hIgG1 AA | 0.2 | Intraperitoneal administration | Once every 3 days for a total of 6 times |
| G2 | 6 | Bispecific antibody 1 | 0.23 | Intraperitoneal administration | Once every 3 days for a total of 6 times |

(continued)

| Group No. | Number of mice per group | Test drug | Dose (mg/kg) | Route of administration | Actual administration frequency and period |
|---|---|---|---|---|---|
| G3 | 6 | Bispecific antibody 1 | 0.05 | Intraperitoneal administration | Once every 3 days for a total of 6 times |
| G4 | 6 | Bispecific antibody 2 | 0.17 | Intraperitoneal administration | Once every 3 days for a total of 6 times |
| G5 | 6 | Bispecific antibody 2 | 0.03 | Intraperitoneal administration | Once every 3 days for a total of 6 times |

The daily behavior of the animals was monitored daily for 16 days after administration. Throughout the experiment, the long and short diameters of the tumor were measured once every 2 days with a vernier caliper. Tumor volume was calculated according to the following formula: tumor volume (mm$^3$) = 0.5 × (long diameter of tumor × short diameter of tumor$^2$). Relative tumor growth inhibition rate TGI (%): TGI% = (1 - T/C) × 100%. T/C % is the relative tumor proliferation rate, i.e., the percentage of the relative tumor volume or tumor weight of the treatment group vs hIgG1 AA control group at a certain time point. T and C are the tumor volume (TV) or tumor weight (TW) at a certain time point for the treatment group and hIgG1 AA control group, respectively. The experimental results for the tumor volume, mouse body weight, tumor weight, and the like of each group of animals were expressed as mean ± standard error (Mean ± SEM). Different treatment groups were compared with the control group for any significant difference by the independent sample T test. Data were analyzed using SPSS. P < 0.05 indicates a significant difference. The experimental results are shown in Table 9 and FIG. 4.

Different dose treatment groups of bispecific antibody 1 and bispecific antibody 2 all showed very significant tumor inhibition effects (p values were all less than 0.001) in the human myeloma NCI-H929 subcutaneous xenograft NPG female mouse MiXeno model, and the tumor growth inhibition rates of bispecific antibody 1 and bispecific antibody 2 both reached 100%. Moreover, the mice showed good tolerance to the test drugs, and no significant weight loss or toxicity occurred in the mice.

Table 9. Tumor volume and tumor growth inhibition rate in each administration group

| Group No. | Test drug | Dose (mg/kg) | Mean tumor volume (mm$^3$) | | T/C (%) | Tumor growth inhibition rate (TGI) (%) | P value |
|---|---|---|---|---|---|---|---|
| | | | Day 0 | Day 16 | | | |
| G1 | hIgG1 AA | 0.2 | 79.33 | 2200.88 | / | / | / |
| G2 | Bispecific antibody 1 | 0.23 | 79.56 | 0.00 | 0.00 | 100 | 4.66e-13*** |
| G3 | Bispecific antibody 1 | 0.05 | 79.14 | 0.00 | 0.00 | 100 | 4.66e-13*** |
| G4 | Bispecific antibody 2 | 0.17 | 79.27 | 0.00 | 0.00 | 100 | 4.66e-13*** |
| G5 | Bispecific antibody 2 | 0.03 | 79.29 | 0.00 | 0.00 | 100 | 4.66e-13*** |

B. Human immune cell reconstitution mouse model, with tumor cells being RPMI8226

The experimental animals were all housed in separate ventilated boxes with constant temperature and humidity. The feeding room was maintained at a temperature of 20.0-26.0°C, with humidity of 30-70% and a light/dark cycle of 12 h/12 h.

RPMI8226 cells were cultured in an RPMI 1640 culture medium containing 10% fetal bovine serum. RPMI8226 cells in the exponential growth phase were collected and resuspended in 0.1 mL of suspension of PBS and matrigel (1:1), and 5 × 10$^6$ RPMI8226 cells were inoculated into the upper right dorsum of the experimental mice. The growth of the tumors was observed periodically. When the mean volume of the tumors reached 76 mm$^3$, the mice were randomly grouped for administration according to the tumor size and the mouse body weight.

Donor PBMCs were purchased from AllCells, LLC. On the day of RPMI8226 cell inoculation, each mouse was intraperitoneally injected with 1 × 10$^7$ PBMCs/0.1 mL PBS to establish a human immune cell reconstitution mouse model.

Before the start of administration, all the animals were weighed, and the tumor volume was measured with a vernier caliper. Given that tumor volume affects the effectiveness of treatment, the mice were grouped using the random grouping design based on their tumor volumes to ensure similar tumor volumes across different groups. Grouping was performed using StudyDirectorTM (version 3.1.399.19, supplier: Studylog System, Inc., S. San Francisco, CA, USA). The day of mouse grouping was recorded as day 0, and intraperitoneal injection was performed once every three days for 6 times starting from D0. The experimental groups and administration regimens for the hIgG1 AA negative control group (purchased from Biointron, Cat. No. B109802) and the test bispecific antibody molecules are shown in Table 10.

Table 10. Experimental groups and administration regimens

| Group No. | Number of mice per group | Test drug | Dose (mg/kg) | Route of administration | Actual administration frequency and period |
|---|---|---|---|---|---|
| G1 | 6 | hIgG1AA | 1 | Intraperitoneal administration | Once every 3 days for a total of 6 times |
| G2 | 6 | Bispecific antibody 1 | 1 | Intraperitoneal administration | Once every 3 days for a total of 6 times |
| G3 | 6 | Bispecific antibody 1 | 0.2 | Intraperitoneal administration | Once every 3 days for a total of 6 times |
| G4 | 6 | Bispecific antibody 2 | 0.73 | Intraperitoneal administration | Once every 3 days for a total of 6 times |
| G5 | 6 | Bispecific antibody 2 | 0.15 | Intraperitoneal administration | Once every 3 days for a total of 6 times |

The daily behavior of the animals was monitored daily for 18 days after administration. Throughout the experiment, the long and short diameters of the tumor were measured twice weekly with a vernier caliper. Tumor volume was calculated according to the following formula: tumor volume ($mm^3$) = $0.5 \times$ (long diameter of tumor $\times$ short diameter of tumor$^2$). Relative tumor growth inhibition rate TGI (%): TGI% = (1 - T/C) $\times$ 100%. T/C % is the relative tumor proliferation rate, i.e., the percentage of the relative tumor volume or tumor weight of the treatment group vs hIgG1 AA control group at a certain time point. T and C are the tumor volume (TV) or tumor weight (TW) at a certain time point for the treatment group and hIgG1 AA control group, respectively. The experimental results for the tumor volume, mouse body weight, tumor weight, and the like of each group of animals were expressed as mean $\pm$ standard error (Mean $\pm$ SEM). Different treatment groups were compared with the control group for any significant difference by the independent sample T test. Data were analyzed using SPSS. P < 0.05 indicates a significant difference. The experimental results are shown in Table 11 and FIG. 5.

Different dose treatment groups of bispecific antibody 1 and bispecific antibody 2 all showed very significant tumor inhibition effects (p values were all less than 0.005) in the human myeloma RPMI8226 subcutaneous xenograft NPG female mouse MiXeno model. Moreover, the mice showed good tolerance to the test drugs, and no significant weight loss or toxicity occurred in the mice.

Table 11. Tumor volume and tumor growth inhibition rate in each administration group

| Group No. | Test drug | Dose (mg/kg) | Mean tumor volume ($mm^3$) | | T/C (%) | Tumor growth inhibition rate (TGI) (%) | P value |
|---|---|---|---|---|---|---|---|
| | | | Day 0 | Day 18 | | | |
| G1 | hIgG1 AA | 1 | 76.13 | 361.78 | / | / | / |
| G2 | Bispecific antibody 1 | 1 | 76.21 | 20.61 | 5.70 | 94.30 | 0.0000031*** |
| G3 | Bispecific antibody 1 | 0.2 | 76.11 | 83.79 | 23.16 | 76.84 | 0.000543*** |
| G4 | Bispecific antibody 2 | 0.73 | 76.09 | 8.09 | 2.24 | 97.76 | 7.33e-7*** |
| G5 | Bispecific antibody 2 | 0.15 | 76.17 | 79.76 | 22.05 | 77.95 | 0.00454** |

## Example 9. Acquisition of Mouse Hybridoma-Derived Anti-GPRC5D Antibodies

Construction of cell lines expressing human GPRC5D, human GPRC5A, and cynomolgus monkey GPRC5D.

A nucleotide sequence encoding human GPRC5D (Uniprot ID: Q9NZD1) was cloned into a pCMV3 vector (SinoBiological, Cat. No. CV011) to obtain a vector for human GPRC5D cell line construction. The obtained vector was transfected into CHOK1 cells (ATCC, Cat. No. CCL-61) to obtain a CHOK1 cell line expressing human GPRC5D (referred to as human GPRC5D-CHOK1 cell line).

A nucleotide sequence encoding cynomolgus monkey GPRC5D (Uniprot ID: A0A2K5W6I2) was cloned into a pCMV3 vector to obtain a vector for cynomolgus monkey GPRC5D cell line construction. The obtained vector was transfected into CHOK1 cells to obtain a CHOK1 cell line expressing cynomolgus monkey GPRC5D (referred to as cynomolgus monkey GPRC5D-CHOK1 cell line).

A nucleotide sequence encoding human GPRC5A (Uniprot ID: Q8NFJ5) was cloned into a pCMV3 vector to obtain a vector for human GPRC5A cell line construction. The obtained vector was transfected into CHOK1 cells to obtain a CHOK1 cell line expressing human GPRC5A (referred to as human GPRC5A-CHOK1 cell line).

The anti-GPRC5D monoclonal antibodies were produced by immunizing mice.

Female Swiss Webster white mice aged 6 weeks (Charles River) were used in the experiment. Housing environment: SPF grade. The purchased mice were housed in a laboratory environment for 1 week, with a light/dark cycle of 12 h/12 h, a temperature of 20-25°C, and humidity of 40-60%. The immunogen was a full-length human GPRC5D plasmid, and each immunization with 4 μg of the plasmid was performed on day 0, day 14, day 28, and day 42. The booster immunization was performed with CHO-K1 cells highly expressing human GPRC5D 4 days prior to splenocyte fusion. Meanwhile, the mouse serum was detected by FACS to determine the antibody titer in the mouse serum. After the booster immunization, mice with high serum antibody titers that tended to plateau were selected for splenocyte fusion. An optimized electrofusion procedure was used to fuse spleen lymphocytes with the myeloma Sp2/0 cells (ATCC® CRL-8287™) to obtain hybridoma cells.

After the fused hybridoma cells were cultured for 7-14 days, the culture supernatant was taken, and the hybridoma supernatant was subjected to antibody screening using CHO-K1 cells highly expressing human GPRC5D in an FACS experiment. The obtained positive antibody strains were further screened using CHO-K1 cells highly expressing cynomolgus monkey GPRC5D, followed by comparison with blank CHO-K1 cells to exclude antibody hybridoma strains with non-specific binding. Screening was performed using the flow cytometry sorting method, and further exclusion screening was performed using CHO-K1 cells highly expressing human GPRC5A, thereby selecting hybridomas specifically binding to human/cynomolgus monkey GPRC5D but not binding to human GPRC5A. Hybridoma cells at the logarithmic growth phase were collected, and the RNA was extracted using Trizol (Invitrogen, 15596-018) and reverse transcribed (PrimeScript™ Reverse Transcriptase, Takara, Cat. No. 2680A). The cDNA obtained by reverse transcription was amplified by PCR using mouse Ig-Primer Set (Novagen, TB326 Rev.B 0503) and then sent for sequencing to obtain 11 monoclonal antibodies: 18CH-1, 18CH-2C, 18CH-3, 18CH-4, 18CH-5, 18CH-7A, 18CH-9, 18CH-10A, 18CH-15, 18CH-16, and 18CH-18, and the amino acid sequences of the variable regions of 18CH-16 are shown in Table 12.

Table 12. Amino acid sequences of monoclonal mouse hybridoma-derived anti-GPRC5D antibody variable regions

| Clone No. | | Heavy chain variable region (VH) | Light chain variable region (VL) |
|---|---|---|---|
| | SEQ ID NO | 27 | 28 |
| 18CH-16 | Amino acid sequence | EVQLQQSGPELVKPGASVKMSCKASG YTFTSYIIHWVKQKPGQGLEWIGYINP YNDGTKYNEKFKGKATLTSDRSSSTA YMEVSSLTSEDSAVYYCARGGIRAFM NYWGQGTSVTVSS | DIVMTQSHKFMSTSVGDRVSITC KASQDVGTAVAWYQQKPGQSPK LLIYWASTRHTGVPDRFTGSGSGT DFTLSISNVQSEDLADYFCQHYIS WTFGGGTNLEIK |

EP 4 714 974 A1

On the basis of the amino acid sequences described above, the CDRs and FRs of the antibody variable regions were defined using the Kabat numbering scheme. The composition of the 6 CDR sequences of 18CH-16 is shown in Table 13 below.

Table 13. CDR sequences of monoclonal mouse hybridoma-derived anti-GPRC5D antibody

| Clone No. | | CDR1 | | CDR2 | | CDR3 | |
|---|---|---|---|---|---|---|---|
| | | SEQ ID NO | Amino acid sequence | SEQ ID NO | Amino acid sequence | SEQ ID NO | Amino acid sequence |
| 18CH-16 | Heavy chain | 11 | SYIIH | 12 | YINPYNDGTKYNEKFKG | 13 | GGIRAFMNY |
| | Light chain | 14 | KASQDVGTAVA | 15 | WASTRHT | 16 | QHYISWT |

## Example 10. Construction of Chimeric Mouse Hybridoma-Derived Anti-GPRC5D Antibodies and Transient Transfection and Expression of Chimeric Mouse Hybridoma-Derived Anti-GPRC5D Antibodies in Eukaryotic Cells

The nucleic acid sequences encoding the heavy chain variable regions and the light chain variable regions of the monoclonal antibodies of the present disclosure obtained after sequencing were separately spliced with the nucleic acid sequences encoding the IgG1 heavy chain constant regions and the κ light chain constant regions to generate target gene fragments, which were then cloned into pTT5 expression vectors to prepare transfection-grade expression plasmids.

Expi293F™ cells (Thermo Fisher Scientific) were cultured in a serum-free culture medium, seeded in a shake flask (Corning Inc.), and cultured on a shaker at 37°C with 8% $CO_2$. The cell density was adjusted, and the recombinant expression vectors containing the target gene fragments and the PEI transfection reagent were mixed according to a suitable ratio and added to a cell culture shake flask. After 6 days of cell culture, the expression supernatant was collected, centrifuged at high speed to remove cell debris, and subjected to affinity purification using a Protein A column. The column was rinsed with PBS until the A280 reading dropped to the baseline. The target protein was eluted with acidic eluent of pH 3.0-pH 3.5, and neutralized with 1 M Tris-HCl (pH 8.0-9.0). After the elution sample was properly concentrated, the elution sample was buffer-exchanged into PBS and aliquoted for later use. The final purified chimeric antibodies were subjected to SDS-PAGE and HPLC for purity analysis and A280 concentration determination.

## Example 11. Binding of Chimeric Mouse Hybridoma-Derived Anti-GPRC5D Antibodies to Cells Expressing GPRC5D

The culture media for both human GPRC5D-CHOK1 cells and cynomolgus monkey GPRC5D-CHOK1 cells were F12K + 10% FBS + 400 μg/mL Hygromycin, and the cells were cultured in a T75 cell culture flask in an incubator at 37°C with 5% $CO_2$. When in use, the cells were washed twice with sterile DPBS and digested with 0.25% trypsin EDTA for about 5 min, and then the digestion was terminated with a complete culture medium.

The resulting cells were centrifuged at 1000 rpm and room temperature for 5 min, and the supernatant was discarded. The cells were resuspended in 100 μL of 1% BSA (in PBS). The cells were counted, and the cell density was adjusted to 1E6 cells/mL. The cells were plated in a 96-well round-bottom culture plate (Corning 3799) and centrifuged at 1500 rpm and 4°C for 5 min, and the supernatant was discarded. The cells were resuspended in 200 μL of 1% BSA (in PBS) and centrifuged again at 1500 rpm and 4°C for 5 min, and the supernatant was discarded. The cells were left to stand at 4°C for later use. The antibody samples to be tested were diluted with 1% BSA (in PBS) at an initial concentration of 100 nM and diluted in 10-fold dilution series to obtain 7 concentrations. The diluted antibody was added at 100 μL/well to resuspend the cells, and the mixture was incubated at 4°C for 1 h. The mixture was centrifuged at 1500 rpm and 4°C for 5 min, and the supernatant was discarded. The cells were resuspended in and washed with 160 μL of 1% BSA (in PBS) and centrifuged at 1500 rpm and 4°C for 5 min, and the supernatant was discarded. The secondary antibody (goat anti human IgG Fc PE) was diluted with 1% BSA (in PBS) at a ratio of 1:400 according to the instructions. The diluted secondary antibody was added at 100 μL/well to resuspend the cells, and the mixture was incubated at 4°C for 0.5 h. The mixture was centrifuged at 1500 rpm and 4°C for 5 min, and the supernatant was discarded. The cells were resuspended in and washed with 200 μL of 1% BSA (in PBS) and centrifuged at 1500 rpm and 4°C for 5 min, and the supernatant was discarded. The cells were resuspended in 100 μL of 1% BSA (in PBS) and filtered through 300-mesh gauze, and the mean fluorescence intensity in the PE channel was measured using a flow cytometer.

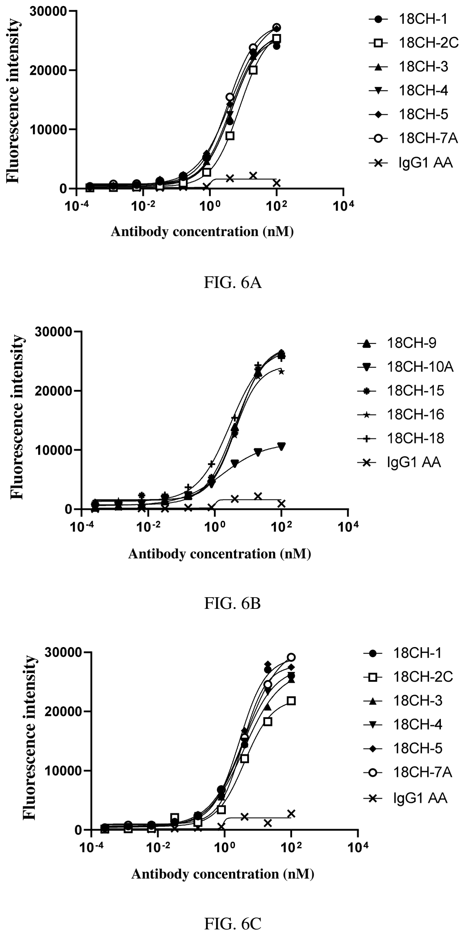

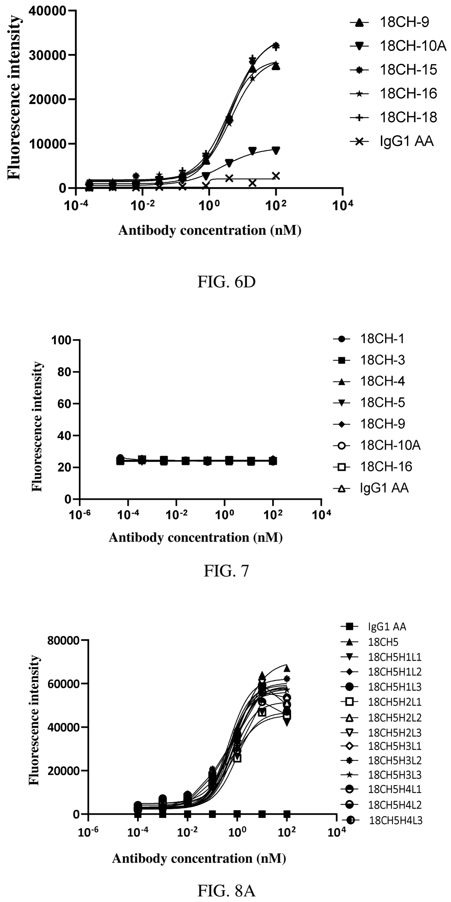

An FCS file was exported from the flow cytometer, and the PE channel mean fluorescence intensity (hereinafter referred to as MFI) of each sample was analyzed by using flowjo software. The analyzed mean fluorescence intensity was imported into Graphpad to analyze the half maximal binding concentration ($EC_{50}$) and the top mean fluorescence intensity (Top MFI) of the antibodies to the cells. The results are shown in Table 14 and FIGs. 6A-6D. The screened chimeric anti-GPRC5D antibodies exhibited relatively good binding to both human GPRC5D and cynomolgus monkey GPRC5D at the cellular level.

Table 14. Binding of chimeric mouse hybridoma-derived anti-GPRC5D antibodies to cells expressing GPRC5D

| Clone No. | Human GPRC5D-CHOK1 | | Cynomolgus monkey GPRC5D CHOK1 | |
|---|---|---|---|---|
| | $EC_{50}$ (nM) | Top mean fluorescence intensity (Top MFI) | $EC_{50}$ (nM) | Top mean fluorescence intensity (Top MFI) |
| IgG1 AA | Not fitted | 1612 | Not fitted | 2041 |
| 18CH-16 | 3.89 | 24304 | 4.39 | 29337 |

## Example 12. Binding of Chimeric Mouse Hybridoma-Derived Anti-GPRC5D Antibodies to GPRC5A of the Same Family

The culture medium for human GPRC5A-CHOK1 cells was F12K + 10% FBS + 400 $\mu$g/mL Hygromycin, and the cells were cultured in a T75 cell culture flask in an incubator at 37°C with 5% $CO_2$. When in use, the cells were washed twice with sterile DPBS and digested with 0.25% trypsin EDTA for about 5 min, and then the digestion was terminated with a complete culture medium.

The resulting cells were centrifuged at 1000 rpm and room temperature for 5 min, and the supernatant was discarded. The cells were resuspended in 100 $\mu$L of 1% BSA (in PBS). The cells were counted, and the cell density was adjusted to 1E6 cells/mL. The cells were plated in a 96-well round-bottom culture plate (Corning 3799) and centrifuged at 1500 rpm and 4°C for 5 min, and the supernatant was discarded. The cells were resuspended in 200 $\mu$L of 1% BSA (in PBS) and centrifuged again at 1500 rpm and 4°C for 5 min, and the supernatant was discarded. The cells were left to stand at 4°C for later use. The antibody samples to be tested were diluted with 1% BSA (in PBS) at an initial concentration of 100 nM and diluted in 10-fold dilution series to obtain 7 concentrations. The diluted antibody was added at 100 $\mu$L/well to resuspend the cells, and the mixture was incubated at 4°C for 1 h. The mixture was centrifuged at 1500 rpm and 4°C for 5 min, and the supernatant was discarded. The cells were resuspended in and washed with 160 $\mu$L of 1% BSA (in PBS) and centrifuged at 1500 rpm and 4°C for 5 min, and the supernatant was discarded. The secondary antibody (goat anti human IgG Fc PE) was diluted with 1% BSA (in PBS) at a ratio of 1:400 according to the instructions. The diluted secondary antibody was added at 100 $\mu$L/well to resuspend the cells, and the mixture was incubated at 4°C for 0.5 h. The mixture was centrifuged at 1500 rpm and 4°C for 5 min, and the supernatant was discarded. The cells were resuspended in and washed with 200 $\mu$L of 1% BSA (in PBS) and centrifuged at 1500 rpm and 4°C for 5 min, and the supernatant was discarded. The cells were resuspended in 100 $\mu$L of 1% BSA (in PBS) and filtered through 300-mesh gauze, and the mean fluorescence intensity in the PE channel was measured using a flow cytometer.

An FCS file was exported from the flow cytometer, and the PE channel mean fluorescence intensity (hereinafter referred to as MFI) of each sample was analyzed by using flowjo software. The analyzed mean fluorescence intensity was imported into Graphpad to analyze the half maximal binding concentration ($EC_{50}$) and the top mean fluorescence intensity (Top MFI) of the antibodies to the cells. The results are shown in FIG. 7, indicating that none of the tested antibodies non-specifically bound to GPRC5A, a member of the same family.

## Example 13. Humanization Design for Mouse Hybridoma-Derived Anti-GPRC5D Antibodies

The 11 chimeric antibodies were subjected to expression and purification tests, cell-level binding tests, and the like, and 2 clones were further selected for humanization design.

Humanization of the monoclonal murine anti-human GPRC5D antibodies was performed as described in many publications in the art. Briefly, human constant domains were used in place of parent (murine antibody) constant domains, and human germline antibody sequences were selected, on the basis of the homology of the murine and human antibodies. On the basis of the typical structure of the murine antibody VH/VL CDR obtained, the heavy chain and light chain variable region sequences were compared with a human antibody germline database to obtain a human germline template with high homology.

The CDR regions of the murine antibodies were grafted onto the selected corresponding humanization template to replace the humanized variable regions, followed by recombination with IgG constant regions (preferably IgG1 for the heavy chain and $\kappa$ for the light chain). Then, on the basis of the three-dimensional structure of the murine antibodies, embedded residues, residues directly interacting with the CDR regions, and residues having an important influence on the conformation of VL and VH were back mutated to design antibodies composed of a plurality of humanized light and heavy chain variable region sequences, wherein 18CH16H2L1 is shown in Table 15.

Table 15. Amino acid sequences of humanized mouse hybridoma-derived antibody (18CH16H2L1) variable regions

| Clone No. | | Heavy chain variable region (VH) | Light chain variable region (VL) |
|---|---|---|---|
| 18CH16H2L1 | SEQ ID NO | 9 | 10 |
| | Amino acid se-quence | QVQLVQSGAEVKKPGASVKVSCK ASGYTFTSYIIHWVRQAPGQGLEW IGYINPYNDGTKYNEKFKGRVTMT SDTSTSTVYMELSSLRSEDTAVYY CARGGIRAFMNYWGQGTLVTVSS | DIQMTQSPSSLSASVGDRVTITC KASQDVGTAVAWYQQKPGKAP KLLIYWASTRHTGVPSRFSGSG SGTDFTLTISSLQPEDFATYYCQ HYISWTFGQGTKVEIK |

## Example 14. Preparation of Humanized Mouse Hybridoma-Derived Anti-GPRC5D Antibodies

Referring to Example 10, the nucleic acid sequences encoding the heavy chain variable regions and the light chain variable regions of the humanized antibodies were separately spliced with the nucleic acid sequences encoding the IgG1 heavy chain constant regions and the x light chain constant regions to generate target gene fragments, which were then cloned into pTT5 expression vectors to prepare transfection-grade expression plasmids.

Expi293F™ cells (Thermo Fisher Scientific) were cultured in a serum-free culture medium, seeded in a shake flask (Corning Inc.), and cultured on a shaker at 37°C with 8% $CO_2$. The cell density was adjusted, and the recombinant expression vectors containing the target gene fragments and the PEI transfection reagent were mixed according to a suitable ratio and added to a cell culture shake flask. After 6 days of cell culture, the expression supernatant was collected, centrifuged at high speed to remove cell debris, and subjected to affinity purification using a Protein A column. The column was rinsed with PBS until the A280 reading dropped to the baseline. The target protein was eluted with acidic eluent of pH 3.0-pH 3.5, and neutralized with 1 M Tris-HCl (pH 8.0-9.0). After the elution sample was properly concentrated, the elution sample was buffer-exchanged into PBS and aliquoted for later use. The final purified humanized antibodies were subjected to SDS-PAGE and HPLC for purity analysis and A280 concentration determination.

## Example 15. Binding of Humanized Mouse Hybridoma-Derived Anti-GPRC5D Antibodies to Cells Expressing GPRC5D

The culture medium for human GPRC5D-CHOK1 cells was F12K + 10% FBS + 400 μg/mL Hygromycin, and the culture medium for NCI-H929 cells was RPMI 1640 + 10% FBS + 0.05 mM mercaptoethanol. The cells were cultured in a T75 cell culture flask in an incubator at 37°C with 5% $CO_2$. When in use, the human GPRC5D-CHOK1 cells were washed twice with sterile DPBS and digested with 0.25% trypsin EDTA for about 5 min, and then the digestion was terminated with a complete culture medium. When in use, the NCI-H929 cells were directly transferred to a 50 mL centrifuge tube using a pipette.

The resulting cells were centrifuged at 1000 rpm and room temperature for 5 min, and the supernatant was discarded. The cells were resuspended in 100 μL of 1% BSA (in PBS). The cells were counted, and the cell density was adjusted to 1E6 cells/mL. The cells were plated in a 96-well round-bottom culture plate (Corning 3799) and centrifuged at 1500 rpm and 4 °C for 5 min, and the supernatant was discarded. The cells were resuspended in 200 μL of 1% BSA (in PBS) and centrifuged again at 1500 rpm and 4 °C for 5 min, and the supernatant was discarded. The cells were left to stand at 4°C for later use. The antibody samples to be tested were diluted with 1% BSA (in PBS) at an initial concentration of 100 nM and diluted in 10-fold dilution series to obtain 7 concentrations. The diluted antibody was added at 100 μL/well to resuspend the cells, and the mixture was incubated at 4°C for 1 h. The mixture was centrifuged at 1500 rpm and 4°C for 5 min, and the supernatant was discarded. The cells were resuspended in and washed with 160 μL of 1% BSA (in PBS) and centrifuged at 1500 rpm and 4 °C for 5 min, and the supernatant was discarded. The secondary antibody (goat anti human IgG Fc PE) was diluted with 1% BSA (in PBS) at a ratio of 1:400 according to the instructions. The diluted secondary antibody was added at 100 μL/well to resuspend the cells, and the mixture was incubated at 4°C for 0.5 h. The mixture was centrifuged at 1500 rpm and 4°C for 5 min, and the supernatant was discarded. The cells were resuspended in and washed with 200 μL of 1% BSA (in PBS) and centrifuged at 1500 rpm and 4 °C for 5 min, and the supernatant was discarded. The cells were resuspended in 100 μL of 1% BSA (in PBS) and filtered through 300-mesh gauze, and the mean fluorescence intensity in the PE channel was measured using a flow cytometer.

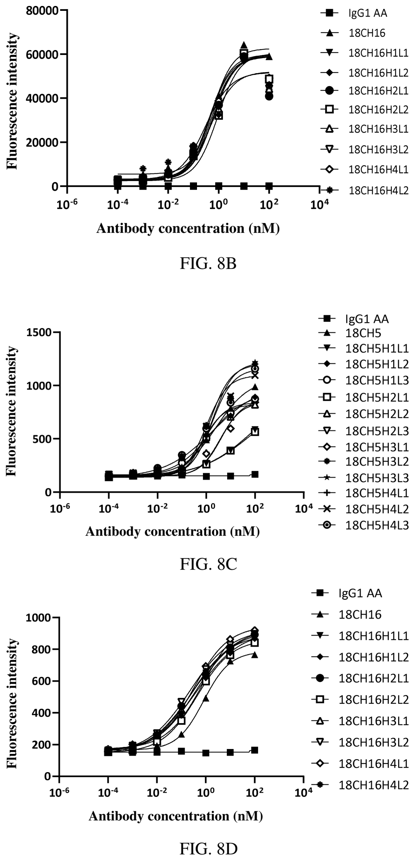

An FCS file was exported from the flow cytometer, and the PE channel mean fluorescence intensity (hereinafter referred to as MFI) of each sample was analyzed by using flowjo software. The analyzed mean fluorescence intensity was imported into Graphpad to analyze the half maximal binding concentration ($EC_{50}$) and the top mean fluorescence intensity (Top MFI) of the antibodies to the cells. The results are shown in Table 16 and FIGs. 8A-8D. The binding of the humanized clone 18CH16 antibody to human GPRC5D-CHOK1 cells and NCI-H929 tumor cells was substantially comparable to that of the parent antibody to human GPRC5D-CHOK1 cells and NCI-H929 tumor cells.

Table 16. Binding of humanized mouse hybridoma-derived anti-GPRC5D antibody 18CH16H2L1 to cells expressing GPRC5D

| Clone No. | Human GPRC5D-CHOK1 | | NCI-H929 | |
|---|---|---|---|---|
| | $EC_{50}$ (nM) | Top mean fluorescence intensity (Top MFI) | $EC_{50}$ (nM) | Top mean fluorescence intensity (Top MFI) |
| IgG1 AA | Not fitted | 51 | Not fitted | 165 |
| 18CH16H2L1 | 0.51 | 58899 | 0.30 | 938 |

The embodiments of the present disclosure described above are merely exemplary, and any person skilled in the art can recognize or determine numerous equivalents of specific compounds, materials, and operations without the need for undue experimentation. All such equivalents are intended to be within the scope of the present disclosure, and are encompassed by the claims.

**Claims**

1. A bispecific antigen-binding molecule, comprising:

   a first polypeptide comprising: (i) an antigen-binding fragment Fab heavy chain domain capable of specifically binding to a first antigen, (ii) a single-chain variable fragment (scFv) domain capable of specifically binding to a second antigen, and (iii) a first Fc domain,
   a second polypeptide comprising: an antigen-binding fragment Fab light chain domain capable of specifically binding to the first antigen, and
   a third polypeptide comprising: a second Fc domain,
   wherein the antigen-binding fragment Fab heavy chain domain of the first polypeptide and the antigen-binding fragment Fab light chain domain of the second polypeptide form a first binding site to the first antigen, the single-chain variable fragment (scFv) domain forms a second binding site to the second antigen, and the first Fc domain and the second Fc domain are associated with each other;
   optionally, the bispecific antigen-binding molecule further comprises a fourth polypeptide, wherein the fourth polypeptide comprises an antigen-binding fragment Fab light chain domain identical to the antigen-binding fragment Fab light chain domain of the second polypeptide and capable of specifically binding to the first antigen, and the third polypeptide further comprises an antigen-binding fragment Fab heavy chain domain capable of specifically binding to the first antigen, wherein the antigen-binding fragment Fab heavy chain domain is identical to the antigen-binding fragment Fab heavy chain domain of the first polypeptide, the C-terminus of the antigen-binding fragment Fab heavy chain domain is linked to the N-terminus of the second Fc domain, and the antigen-binding fragment Fab heavy chain domain of the third polypeptide and the antigen-binding fragment Fab light chain domain of the fourth polypeptide form a third binding site to the first antigen.

2. The bispecific antigen-binding molecule according to claim 1, wherein the single-chain variable fragment (scFv) domain comprises a heavy chain variable region and a light chain variable region;

   preferably, the heavy chain variable region of the single-chain variable fragment (scFv) domain is linked to the light chain variable region of the single-chain variable fragment (scFv) domain via a first linker, wherein the C-terminus of the heavy chain variable region of the single-chain variable fragment (scFv) domain is fused to the N-terminus of the first linker, and the C-terminus of the first linker is fused to the N-terminus of the light chain variable region of the single-chain variable fragment (scFv) domain;
   more preferably, the first linker comprises the amino acid sequence $(G_4S)_n$, wherein n is any integer of 1-10.

3. The bispecific antigen-binding molecule according to claim 1 or 2, wherein the first Fc domain comprises a first CH2 domain and a first CH3 domain of an immunoglobulin, wherein the C-terminus of the first CH2 domain is fused to the N-terminus of the first CH3 domain; the second Fc domain comprises a second CH2 domain and a second CH3 domain of an immunoglobulin, wherein the C-terminus of the second CH2 domain is fused to the N-terminus of the second CH3 domain;

preferably, the first CH3 domain comprises a "knob" structure, and the second CH3 domain comprises a "hole" structure; more preferably, the "knob" structure comprises amino acid substitutions S354C and T366W, and the "hole" structure comprises amino acid substitutions Y349C, T366S, L368A, and Y407V;
preferably, the Fc domain is derived from IgG1;
preferably, the single-chain variable fragment (scFv) domain is linked to the first Fc domain via a second linker, wherein the C-terminus of the single-chain variable fragment (scFv) domain is fused to the N-terminus of the second linker, and the C-terminus of the second linker is fused to the N-terminus of the first Fc domain;
more preferably, the second linker comprises the amino acid sequence EPKSS.

4. The bispecific antigen-binding molecule according to any one of claims 1-3, wherein the antigen-binding fragment Fab heavy chain domain comprises a heavy chain variable region and a CH1 domain of an immunoglobulin, wherein the C-terminus of the heavy chain variable region is fused to the N-terminus of the CH1 domain; the antigen-binding fragment Fab light chain domain comprises a light chain variable region and a light chain constant region of an immunoglobulin, wherein the C-terminus of the light chain variable region is fused to the N-terminus of the light chain constant region;

preferably, the antigen-binding fragment Fab heavy chain domain of the first polypeptide is linked to the single-chain variable fragment (scFv) domain via a third linker, wherein the C-terminus of the antigen-binding fragment Fab heavy chain domain is fused to the N-terminus of the third linker, and the C-terminus of the third linker is fused to the N-terminus of the single-chain variable fragment (scFv) domain;
more preferably, the third linker comprises the amino acid sequence $(G_4S)_n$, wherein n is any integer of 1-10;
optionally, the C-terminus of the antigen-binding fragment Fab heavy chain domain of the third polypeptide is linked to the N-terminus of the second Fc domain via a fourth linker.

5. The bispecific antigen-binding molecule according to any one of claims 1-4, wherein the first polypeptide comprises the following structure: Fab heavy chain domain-third linker-scFv domain-second linker-first Fc domain, preferably, the first polypeptide comprises the following structure: Fab heavy chain variable region-Fab CH1-third linker-scFv heavy chain variable region-first linker-scFv light chain variable region-second linker-first CH2-first CH3; the second polypeptide comprises the following structure: Fab light chain variable region-light chain constant region; the third polypeptide comprises the following structure: second CH2-second CH3;
optionally, the third polypeptide comprises the following structure: Fab heavy chain domain-second Fc domain, preferably comprises the following structure: Fab heavy chain variable region-Fab CH1-second CH2-second CH3, and the fourth polypeptide comprises the following structure: Fab light chain variable region-light chain constant region.

6. The bispecific antigen-binding molecule according to any one of claims 1-5, comprising one or more amino acid substitutions selected from the following group: L234A, L235A, and H435R, wherein, preferably, the L234A and L235A are substitutions on the first polypeptide and/or the third polypeptide; preferably, the H435R substitution is a substitution on the third polypeptide.

7. The bispecific antigen-binding molecule according to any one of claims 1-6, wherein the second antigen is CD3, preferably CD3ε;

preferably, the single-chain variable fragment (scFv) domain comprises an HCDR1 set forth in SEQ ID NO: 19, an HCDR2 set forth in SEQ ID NO: 20, an HCDR3 set forth in SEQ ID NO: 21, an LCDR1 set forth in SEQ ID NO: 22, an LCDR2 set forth in SEQ ID NO: 23, and an LCDR3 set forth in SEQ ID NO: 24;
more preferably, the single-chain variable fragment (scFv) domain comprises a heavy chain variable region set forth in SEQ ID NO: 17 and a light chain variable region set forth in SEQ ID NO: 18;
more preferably, the single-chain variable fragment (scFv) domain comprises the amino acid sequence set forth in SEQ ID NO: 8.

8. The bispecific antigen-binding molecule according to any one of claims 1-7, wherein the first Fc domain comprises the amino acid sequence set forth in SEQ ID NO: 26, and the second Fc domain comprises the amino acid sequence set forth in SEQ ID NO: 4.

9. The bispecific antigen-binding molecule according to any one of claims 1-8, wherein the first antigen is GPRC5D;

preferably, the antigen-binding fragment Fab heavy chain domain comprises an HCDR1 set forth in SEQ ID NO: 11, an HCDR2 set forth in SEQ ID NO: 12, and an HCDR3 set forth in SEQ ID NO: 13; the antigen-binding fragment Fab light chain domain comprises an LCDR1 set forth in SEQ ID NO: 14, an LCDR2 set forth in SEQ ID NO: 15, and an LCDR3 set forth in SEQ ID NO: 16;

more preferably, the antigen-binding fragment Fab heavy chain domain comprises a heavy chain variable region set forth in SEQ ID NO: 9; the antigen-binding fragment Fab light chain domain comprises a light chain variable region set forth in SEQ ID NO: 10;

more preferably, the antigen-binding fragment Fab heavy chain domain comprises the amino acid sequence set forth in SEQ ID NO: 25; the antigen-binding fragment Fab light chain domain comprises the amino acid sequence set forth in SEQ ID NO: 3.

10. The bispecific antigen-binding molecule according to any one of claims 1-9, wherein the first polypeptide comprises the amino acid sequence set forth in SEQ ID NO: 1, the second polypeptide comprises the amino acid sequence set forth in SEQ ID NO: 3, and/or, the third polypeptide comprises the amino acid sequence set forth in SEQ ID NO: 4;

optionally, the first polypeptide comprises the amino acid sequence set forth in SEQ ID NO: 1, the second polypeptide and/or the fourth polypeptide comprise the amino acid sequence set forth in SEQ ID NO: 3, and the third polypeptide comprises the amino acid sequence set forth in SEQ ID NO: 2.

11. A bispecific antigen-binding molecule, wherein an epitope to which the bispecific antigen-binding molecule is capable of binding

is identical to or overlaps with an epitope targeted by an antibody comprising an HCDR1 set forth in SEQ ID NO: 11, an HCDR2 set forth in SEQ ID NO: 12, an HCDR3 set forth in SEQ ID NO: 13, an LCDR1 set forth in SEQ ID NO: 14, an LCDR2 set forth in SEQ ID NO: 15, and an LCDR3 set forth in SEQ ID NO: 16;

preferably, an epitope to which the bispecific antigen-binding molecule is capable of binding

is identical to or overlaps with an epitope targeted by an antibody comprising the heavy chain variable region amino acid sequence set forth in SEQ ID NO: 9 and the light chain variable region amino acid sequence set forth in SEQ ID NO: 10.

12. The bispecific antigen-binding molecule according to claim 11, wherein the bispecific antigen-binding molecule is capable of binding to CD3, preferably is capable of binding to CD3ε.

13. A bispecific antigen-binding molecule, comprising:

(A) a first binding moiety capable of specifically binding to GPRC5D; and
(B) a second binding moiety, wherein an antigen or epitope to which the second binding moiety specifically binds is different from that to which the first binding moiety binds,

wherein the first binding moiety comprises:

an HCDR1 set forth in SEQ ID NO: 11, an HCDR2 set forth in SEQ ID NO: 12, an HCDR3 set forth in SEQ ID NO: 13, an LCDR1 set forth in SEQ ID NO: 14, an LCDR2 set forth in SEQ ID NO: 15, and an LCDR3 set forth in SEQ ID NO: 16;

preferably, the first binding moiety comprises:

a heavy chain variable region set forth in SEQ ID NO: 9 and a light chain variable region set forth in SEQ ID NO: 10.

14. The bispecific antigen-binding molecule according to claim 13, wherein the second binding moiety is capable of binding to CD3, preferably is capable of binding to CD3ε.

15. A GPRC5D-binding molecule or an antigen-binding fragment thereof, comprising: the HCDR sequences of a heavy chain variable region set forth in SEQ ID NO: 9, and/or, the LCDR sequences of a light chain variable region set forth in SEQ ID NO: 10, wherein, preferably, the GPRC5D-binding molecule or the antigen-binding fragment thereof comprises an HCDR1 set forth in SEQ ID NO: 11, an HCDR2 set forth in SEQ ID NO: 12, and an HCDR3 set forth in SEQ ID NO: 13, and/or, an LCDR1 set forth in SEQ ID NO: 14, an LCDR2 set forth in SEQ ID NO: 15, and an LCDR3 set forth in SEQ ID NO: 16;

more preferably, the GPRC5D-binding molecule or the antigen-binding fragment thereof comprises the heavy chain variable region amino acid sequence set forth in SEQ ID NO: 9 and the light chain variable region amino acid sequence set forth in SEQ ID NO: 10;

further preferably, the GPRC5D-binding molecule or the antigen-binding fragment thereof comprises a heavy chain constant region and a light chain constant region; preferably, the GPRC5D-binding molecule or the antigen-binding fragment thereof comprises an IgG1 heavy chain constant region and a κ light chain constant region.

**16.** A nucleic acid molecule, wherein the nucleic acid molecule encodes the bispecific antigen-binding molecule according to any one of claims 1-14 or the GPRC5D-binding molecule or the antigen-binding fragment thereof according to claim 15.

**17.** An expression vector, comprising the nucleic acid molecule according to claim 16.

**18.** A host cell, comprising the nucleic acid molecule according to claim 16 or the expression vector according to claim 17, wherein, preferably, the host cell is a prokaryotic cell or a eukaryotic cell; the prokaryotic cell is preferably *Escherichia coli;* the eukaryotic cell is preferably a mammalian cell or a yeast; more preferably, the mammalian cell is a CHO cell, Expi293, or a HEK293 cell.

**19.** A method for preparing the bispecific antigen-binding molecule according to any one of claims 1-14 or the GPRC5D-binding molecule or the antigen-binding fragment thereof according to claim 15, comprising: culturing the host cell according to claim 18 under suitable conditions.

**20.** An antibody-drug conjugate, wherein the antibody-drug conjugate is formed by conjugating the bispecific antigen-binding molecule according to any one of claims 1-14 or the GPRC5D-binding molecule or the antigen-binding fragment thereof according to claim 15 to an additional bioactive molecule; preferably, the additional bioactive molecule is a small-molecule drug; preferably, the bispecific antigen-binding molecule is linked to the additional bioactive molecule via a linker.

**21.** A pharmaceutical composition, comprising the bispecific antigen-binding molecule according to any one of claims 1-14, the GPRC5D-binding molecule or the antigen-binding fragment thereof according to claim 15, the nucleic acid molecule according to claim 16, the expression vector according to claim 17, the host cell according to claim 18, and/or the antibody-drug conjugate according to claim 20, wherein, preferably, the pharmaceutical composition further comprises a pharmaceutically acceptable carrier and/or one or more additional therapeutic agents.

**22.** Use of the bispecific antigen-binding molecule according to any one of claims 1-14, the GPRC5D-binding molecule or the antigen-binding fragment thereof according to claim 15, the nucleic acid molecule according to claim 16, the expression vector according to claim 17, the host cell according to claim 18, the antibody-drug conjugate according to claim 20, and/or the pharmaceutical composition according to claim 21 in preparing a medicament for treating, alleviating, and/or preventing a tumor,

wherein, preferably, the tumor is a GPRC5D-positive tumor;
preferably, the tumor is selected from: lymphomas such as multiple myeloma, and metastatic cancers of the tumors described above.

**23.** A method for inducing death of a cell expressing GPRC5D *in vivo* or *in vitro,* comprising contacting the cell with the bispecific antigen-binding molecule according to any one of claims 1-14, the GPRC5D-binding molecule or the antigen-binding fragment thereof according to claim 15, the nucleic acid molecule according to claim 16, the expression vector according to claim 17, the host cell according to claim 18, the antibody-drug conjugate according to claim 20, and/or the pharmaceutical composition according to claim 21, wherein, preferably, the cell expressing GPRC5D is a tumor cell;
preferably, the tumor cell is a cell of a tumor selected from the following: lymphomas such as multiple myeloma, and metastatic cancers of the tumors described above.

**24.** A method for treating a disease associated with GPRC5D expression in a subject, comprising administering to a subject in need thereof the bispecific antigen-binding molecule according to any one of claims 1-14, the GPRC5D-binding molecule or the antigen-binding fragment thereof according to claim 15, the nucleic acid molecule according to claim 16, the expression vector according to claim 17, the host cell according to claim 18, the antibody-drug conjugate according to claim 20, and/or the pharmaceutical composition according to claim 21,

wherein, preferably, the disease is a tumor;

preferably, the tumor is lymphoma such as multiple myeloma, or a metastatic cancer of the tumor described above;

preferably, the method further comprises administering to the subject an additional therapeutic agent.

25. The bispecific antigen-binding molecule according to any one of claims 1-14, the GPRC5D-binding molecule or the antigen-binding fragment thereof according to claim 15, the nucleic acid molecule according to claim 16, the expression vector according to claim 17, the host cell according to claim 18, the antibody-drug conjugate according to claim 20, and/or the pharmaceutical composition according to claim 21, for use in therapy.

Bispecific
antibody 1

Bispecific
antibody 2

Anti-GPRC5D full-length
antibody Fab fragment

Anti-GPRC5D antibody heavy chain variable region

Anti-GPRC5D antibody light chain variable region

Anti-CD3 antibody light chain variable region

Anti-CD3 antibody heavy chain variable region

Fc domains with "knob" and "hole" structures

FIG. 1

IgG1 AA
Bispecific antibody 1
Bispecific antibody 2

FIG. 2A

IgG1 AA
Bispecific antibody 1
Bispecific antibody 2

FIG. 2B

FIG. 2C

FIG. 3A

FIG. 3B

FIG. 3C

FIG. 3D

FIG. 3E

FIG. 3F

FIG. 4

FIG. 5

FIG. 6A

FIG. 6B

FIG. 6C

FIG. 6D

FIG. 7

FIG. 8A

FIG. 8B

FIG. 8C

FIG. 8D

# EP 4 714 974 A1

## INTERNATIONAL SEARCH REPORT

| | International application No. |
|---|---|
| | **PCT/CN2024/093243** |

### A. CLASSIFICATION OF SUBJECT MATTER

C07K16/28(2006.01)i; A61K39/395(2006.01)i; A61P35/00(2006.01)i; C07K16/46(2006.01)i; C12N15/13(2006.01)i; C12N15/63(2006.01)i

According to International Patent Classification (IPC) or to both national classification and IPC

### B. FIELDS SEARCHED

Minimum documentation searched (classification system followed by classification symbols)

IPC:C07K16, A61K39, C12N15, A61P35

Documentation searched other than minimum documentation to the extent that such documents are included in the fields searched

Electronic data base consulted during the international search (name of data base and, where practicable, search terms used)

CNTXT, ENTXT, ENTXTC, OETXT, VEN, CJFD, PubMed; ISI web of knowledge: 中国专利生物序列检索系统; Genbank; EMBL-EBI; STN: GPRC5D, CD3, 抗体, antibody, 双特异性, bispecific, SEQ ID NO: 9-16, 多发性骨髓瘤

### C. DOCUMENTS CONSIDERED TO BE RELEVANT

| Category* | Citation of document, with indication, where appropriate, of the relevant passages | Relevant to claim No. |
|---|---|---|
| PX | WO 2023151613 A1 (SHANGHAI QILU PHARMACEUTICAL RESEARCH AND DEVELOPMENT CENTRE LTD.) 17 August 2023 (2023-08-17) see claims 1-12, and description, page 1, paragraph 8 to page 12, paragraph 1 | 1-8, 16-22, 25 |
| X | US 2020087412 A1 (SHANGHAI TONGJI HOSPITAL) 19 March 2020 (2020-03-19) see claims 1-22, and description, paragraphs 50-264 | 1-8, 16-22, 25 |
| X | CN 109715667 A (JANSSEN PHARMACEUTICA NV) 03 May 2019 (2019-05-03) claims 1-65, and description, paragraphs 12-143 | 11, 12, 16-22, 23 (in part), 25 |
| X | CN 115232209 A (NANJING BIOHENG BIOTECH CO., LTD.) 25 October 2022 (2022-10-25) claims 1-27, and description, paragraphs 5-126 | 11, 12, 16-22, 23 (in part), 25 |
| Y | US 2020087412 A1 (SHANGHAI TONGJI HOSPITAL) 19 March 2020 (2020-03-19) see claims 1-22, and description, paragraphs 50-264 | 9, 16-22, 23 (in part), 25 |
| Y | CN 109715667 A (JANSSEN PHARMACEUTICA NV) 03 May 2019 (2019-05-03) claims 1-65, and description, paragraphs 12-143 | 9, 16-22, 23 (in part), 25 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **27 August 2024** | **06 September 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/093243**

### C.  DOCUMENTS CONSIDERED TO BE RELEVANT

| Category* | Citation of document, with indication, where appropriate, of the relevant passages | Relevant to claim No. |
|---|---|---|
| Y | CN 115232209 A (NANJING BIOHENG BIOTECH CO., LTD.) 25 October 2022 (2022-10-25) <br> claims 1-27, and description, paragraphs 5-126 | 9, 16-22, 23 (in part), 25 |

Form PCT/ISA/210 (second sheet) (July 2022)

**INTERNATIONAL SEARCH REPORT**

| International application No. |
| --- |
| **PCT/CN2024/093243** |

| Box No. I       Nucleotide and/or amino acid sequence(s) (Continuation of item 1.c of the first sheet) |
| --- |

1. With regard to any nucleotide and/or amino acid sequence disclosed in the international application, the international search was carried out on the basis of a sequence listing:

     a.   ☑   forming part of the international application as filed.

     b.   ☐   furnished subsequent to the international filing date for the purposes of international search (Rule 13*ter*.1(a)),

            ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
|---|
| **PCT/CN2024/093243** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **23 (in part), 24**
   because they relate to subject matter not required to be searched by this Authority, namely:

   Claim 23 relates to a method for inducing death of cells expressing GPRC5D in vivo, and claim 24 relates to a method for treating diseases related to the expression of GPRC5D in a subject. Claims 23 (in part) and 24 relate to a disease diagnosis and treatment method, which falls under treatment methods for diseases, as well as diagnostic methods, as defined in PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
| --- |
| **PCT/CN2024/093243** |

| Patent document cited in search report | | | Publication date (day/month/year) | Patent family member(s) | | | Publication date (day/month/year) |
| --- | --- | --- | --- | --- | --- | --- | --- |
| WO | 2023151613 | A1 | 17 August 2023 | TW | 202342534 | A | 01 November 2023 |
| US | 2020087412 | A1 | 19 March 2020 | US | 11066476 | B2 | 20 July 2021 |
| CN | 109715667 | A | 03 May 2019 | AR | 109499 | A1 | 19 December 2018 |
| | | | | CR | 20190025 | A | 14 March 2019 |
| | | | | ZA | 201901066 | B | 28 October 2020 |
| | | | | JP | 2024054176 | A | 16 April 2024 |
| | | | | BR | 112019001055 | A2 | 01 October 2019 |
| | | | | NI | 201900005 | A | 10 May 2019 |
| | | | | UY | 37340 | A | 31 January 2018 |
| | | | | TW | 201809005 | A | 16 March 2018 |
| | | | | KR | 20190028534 | A | 18 March 2019 |
| | | | | KR | 102572091 | B1 | 31 August 2023 |
| | | | | MX | 2024001615 | A | 28 February 2024 |
| | | | | PH | 12019500152 | A1 | 14 October 2019 |
| | | | | ECSP | 19012075 | A | 28 February 2019 |
| | | | | EP | 3487882 | A2 | 29 May 2019 |
| | | | | US | 2023227548 | A1 | 20 July 2023 |
| | | | | US | 11884722 | B2 | 30 January 2024 |
| | | | | MX | 2023006449 | A | 15 June 2023 |
| | | | | US | 2023084967 | A1 | 16 March 2023 |
| | | | | US | 11685777 | B2 | 27 June 2023 |
| | | | | CA | 3031472 | A1 | 25 January 2018 |
| | | | | KR | 20230128409 | A | 04 September 2023 |
| | | | | EA | 201990346 | A1 | 28 June 2019 |
| | | | | CL | 2019000146 | A1 | 26 April 2019 |
| | | | | IL | 264334 | A | 28 February 2019 |
| | | | | IL | 264334 | B1 | 01 January 2024 |
| | | | | IL | 264334 | B2 | 01 May 2024 |
| | | | | AU | 2017299673 | A1 | 07 February 2019 |
| | | | | AU | 2017299673 | B2 | 01 August 2024 |
| | | | | IL | 298277 | A | 01 January 2023 |
| | | | | MA | 45712 | A | 29 May 2019 |
| | | | | JP | 2019527061 | A | 26 September 2019 |
| | | | | JP | 7292200 | B2 | 16 June 2023 |
| | | | | WO | 2018017786 | A2 | 25 January 2018 |
| | | | | US | 2018037651 | A1 | 08 February 2018 |
| | | | | US | 10562968 | B2 | 18 February 2020 |
| | | | | JP | 2023011774 | A | 24 January 2023 |
| | | | | JP | 7469432 | B2 | 16 April 2024 |
| | | | | PE | 20190392 | A1 | 13 March 2019 |
| | | | | CO | 2019001114 | A2 | 19 February 2019 |
| | | | | MX | 2019000825 | A | 04 September 2019 |
| | | | | PE | 20240884 | A1 | 24 April 2024 |
| | | | | TW | 781108 | B1 | 21 October 2022 |
| | | | | CN | 116333133 | A | 27 June 2023 |
| CN | 115232209 | A | 25 October 2022 | CN | 115232209 | B | 19 December 2023 |
| | | | | WO | 2022222910 | A1 | 27 October 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310552022
- US 5731168 A
- US 7695936 B

**Non-patent literature cited in the description**

- **RIDGWAY et al.** *Prot Eng*, 1996, vol. 9, 617-621
- **CARTER**. *J Immunol Meth*, 2001, vol. 248, 7-15
- **BRAUNER-OSBORNE, H et al.** *Biochim. Biophys. Acta*, 2001, vol. 1518, 237-248